# EUROPEAN PATENT SPECIFICATION

(11) **EP 4 108 291 B1**
(45) Date of publication and mention of the grant of the patent: **27.11.2024**
(21) Application number: 21216803.3
(22) Date of filing: 22.12.2021
(51) Int. Cl.: A61N 1/36, A61N 1/372

(54) **CONFIGURABLE PATIENT PROGRAMMER FOR NEUROSTIMULATION DEVICE**
KONFIGURIERBARES PATIENTENPROGRAMMIERGERÄT FÜR EINE NEUROSTIMULATIONSVORRICHTUNG
PROGRAMMATEUR PATIENT CONFIGURABLE POUR DISPOSITIF DE NEUROSTIMULATION

(30) Priority: 25.06.2021 US 202117304764
(43) Date of publication of application: 28.12.2022
(73) Proprietor: ONWARD Medical N.V., 5611 ZX Eindhoven (NL)
(72) Inventor: Brouns, Robin, Eindhoven (NL); Caban, Miroslav, Eindhoven (NL); D'Ercole, Marina, Eindhoven (NL)
(74) Representative: DTS Patent- und Rechtsanwälte PartmbB

(56) References cited:
- US-A1- 2002 123 672
- US-A1- 2016 367 827
- US-A1- 2018 133 481

## Description

### BACKGROUND

### FIELD OF THE INVENTION

The present invention relates to a system for rehabilitation or recovery from neurological disorders. The invention also relates to a computer-readable storage medium comprising instructions for assisting and tracking a user with a neurological disorder.

### DISCUSSION OF THE RELATED ART

Recovery from spinal cord injuries or diseases that result in spinal cord dysfunction can be a long arduous process. Both neuromodulation/neurostimulation and physical therapy exercises can be useful tools to aid in this recovery process. Accordingly, one of the key limiting factors to successful spinal cord recovery is poor patient compliance with prescribed physical therapy exercise regimens. Physical therapy exercise can be effectively supervised at various inpatient or outpatient facilities (e.g., rehab centers, physiotherapy practices). However, the length of time between supervised sessions often necessitates the implementation of various at-home exercise regimens to advance the recovery process. Because physical therapy exercises can often be difficult, painful, or otherwise uncomfortable or because of forgetfulness, patients often neglect to fully complete such at-home exercise regimens and may even misrepresent the extent of their at-home compliance to health care providers. Failure to complete physical therapy regimens can lead to prolonged patient recovery. Misinformed healthcare providers can lead to confusion over the best course of action to take in treating the patient. Additionally, even if patients are diligent in their completion of at-home physical therapy exercise regimens, the absence of neuromodulation/neurostimulation means that the patients are not getting the full benefits that physical therapy exercise can provide.

Traditionally, health care providers have attempted to deal with poor patient compliance by scheduling more frequent or additional supervised sessions at rehab facilities or at home with trained caretakers. However, this methodology often leads to a greater financial burden on the patient and greater strains on the health care facilities that often result in increased difficulty of patients to get appointments. Another way in which health care providers have attempted to deal with poor patient compliance is by asking a patient to fill out a daily activity log to record the exercises that are performed, and to show the activity log to the health care provider at the next appointment. However, this methodology places an additional burden on the patient (which given the nature of the neurological disorder may also be very difficult or impossible for the patient to fill out without assistance) and it is still susceptible to patients misrepresenting the extent of their compliance.

US 2018/0133481 discloses an active closed-loop medical system comprising at least one implantable medical device, at least one non-implanted component and at least a controller for controlling the implantable medical device, wherein the implantable medical device, the non-implanted component and the controller are connected for data exchange, wherein the implantable medical device, the non-implanted component and the controller forming in the active state a closed-loop system in such that the implantable medical device is controlled by the controller on the basis of the signals exchanged with the non-implanted component.

In view of the foregoing, it is desirable to improve patient compliance with at-home physical therapy exercise regimens or other prescribed therapy in patients with neurological disorders and to do so without placing undue additional burdens on patients and health care providers. For example, there is a need for an improved method and system to (i) encourage patients complete their prescribed therapy and (ii) allow health care providers to monitor patients' progress.

### SUMMARY OF THE INVENTION

The present invention provides a system for assisting and tracking a user with a neurological disorder as defined in claim 1.

The system of the invention is suitable for use in implementing methods of assisting and tracking a user with a neurological disorder. Exemplary methods are described in the following for the purpose of improving understanding of the invention, but do not form part of the same.

In one example, a non-claimed method, not forming part of the invetnion, for assisting and tracking a user with a neurological disorder includes receiving, from a user interface, a selection of a physical activity that the user wishes to perform; sending a control signal to a neurostimulation device, the control signal including instructions for the neurostimulation device to implement a neurostimulation therapy regimen corresponding to the selected physical activity that the user wishes to perform; receiving an activity feedback signal from at least one sensor, the activity feedback signal including information regarding performance of the selected physical activity when performed by the user; and sending a monitoring signal to a remotely located caregiver device, the monitoring signal including activity data derived from the information regarding performance of the selected physical activity.

According to the invention, a system for assisting and tracking a user with a neurological disorder includes: a user interface configured to allow the user to select an activity that the user wishes to perform; and a controller. The controller is configured to send a control signal to a neurostimulation device, the control signal including instructions for the neurostimulation device to implement a neurostimulation therapy regimen corresponding to the selected activity that the user wishes to perform; receive an activity feedback signal from at least one sensor, the activity feedback signal including information regarding performance of the selected activity when performed by the user; and send a monitoring signal to a remotely located caregiver device, the monitoring signal including activity data derived from the information regarding performance of the selected activity. In particular, said activity is a rehabilitation exercise routine. Activity data includes information regarding how much of the rehabilitation exercise routine was completed by the user.

Further, a computer-readable storage medium having instructions for assisting and tracking a user with a neurological disorder, which when executed on a computer processor causes the processor to perform a method, the method includes: receiving, from a user interface, a selection of an activity that the user wishes to perform; sending a control signal to a neurostimulation device, the control signal including instructions for the neurostimulation device to implement a neurostimulation therapy regimen corresponding to the selected activity that the user wishes to perform; receiving an activity feedback signal from at least one sensor, the activity feedback signal including information regarding performance of the selected activity when performed by the user; and sending a monitoring signal to a remotely located caregiver device, the monitoring signal including activity data derived from the information regarding performance of the selected activity. In particular, said activity is a rehabilitation exercise routine. Activity data includes information regarding how much of the rehabilitation exercise routine was completed by the user.

### BRIEF DESCRIPTION OF THE DRAWINGS

The accompanying drawings, which are incorporated in and constitute a part of this disclosure, illustrate various embodiments and aspects of the present invention. In the drawings:
Fig. 1 illustrates a diagram of a neuromodulation system in accordance with the invention;
Fig. 2 illustrates a diagram of an embodiment of a neuromodulator for use in the neuromodulation system of Fig. 1; and
Fig. 3 is a flowchart for an exemplary non-claimed method for assisting and tracking a user with a neurological disorder, which can be implemented by using the system of the invention.

### DETAILED DESCRIPTION

The following detailed description refers to the accompanying drawings. Wherever possible, the same reference numbers are used in the drawings and in the following description to refer to the same or similar parts. While several exemplary embodiments and features of the invention are described herein, modifications, adaptations, and other implementations are possible. For example, substitutions, additions, or modifications may be made to the components illustrated in the drawings, and the exemplary methods described herein may be modified by substituting, reordering, or adding steps to the disclosed methods.

Systems consistent with the invention generally relate to rehabilitation. More particularly, systems consistent with the invention relate to the rehabilitation or recovery from neurological disorders.

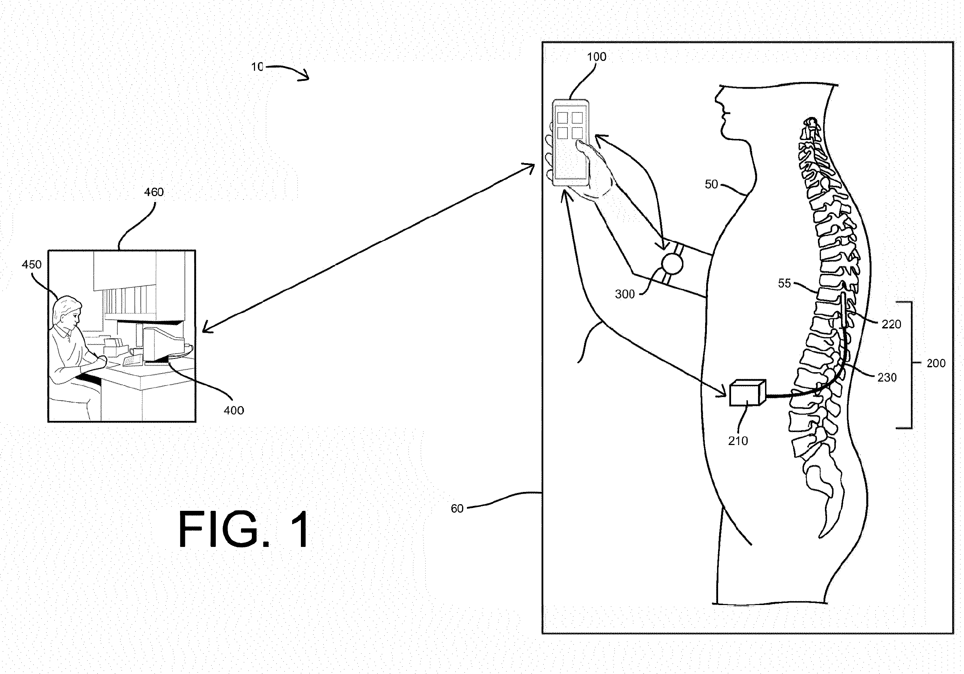

Figure 1 shows a user 50 at an unsupervised location 60 utilizing a neuromodulation system 10 (it is noted that for the purposes of this invention, the term neuromodulation and neurostimulation are equivalent unless context dictates otherwise) to treat a neurological disorder. The neuromodulation system 10 includes a computerized programable device 100 to selectively control neuromodulation stimulation of a neuromodulator 200 that may be implanted on or externally applied to the user's spine 55. At least one sensor 300 may be placed on various parts of the user's 50 body and is configured to send signals regarding the status of the user's 50 body to the programable device 100. A healthcare provider workstation 400 accessible to a healthcare provider 450 is located at a provider location 460 (e.g., at a hospital, clinic, rehab center, etc.) that is located remotely from the user 50 at the unsupervised location 60. The healthcare provider workstation 400 is configured to send and receive signals from the programable device 100 (e.g., via a network, cloud-based service, etc.). It is noted that while Fig. 1 depicts a single user 50 with the programable device 100, there may be several users 50 at different locations 60 each having an individual programable device 100 that are all configured to send and receive signals from the healthcare provider workstation 400.

The programable device 100 and the health care provider workstation 400 may each be a general-purpose computer, a personal computer, a workstation, a mainframe computer, a notebook, a global positioning device, a laptop computer, a smart phone, a smart watch, a tablet, a personal digital assistant, a network server, and any other electronic device that may interact with a user to develop programming code

The neuromodulator 200 may be a neurostimulator 200. The neurostimulator 200 may be or may comprise a pulse generator 210 (e.g., an implantable pulse generator and/or non-implantable pulse generator), an electrode lead 220 having at least one electrode, and at least one wire 230 connecting the electrode lead 220 to the pulse generator 210 and allowing the pulse generator 210 to send electrical pulses to the electrode lead 210.

Examples of the sensors 300 may include one or more of an accelerometer, gyroscope, sphygmomanometer, a force sensor, a pressure sensor, an inertial measurement unit (IMU) sensor, electrocardiography (ECG) sensor, an electroencephalography (EEG) sensor, an electromyography (EMG) sensor, a plethysmography sensor, a thermometer, a pulse oximetry sensor, and any other suitable sensor.

The neuromodulation system 10 can be used for the treatment of dysfunctions, damage, or disorders of the nervous system. Such dysfunctions, damage, or disorders can arise from spinal cord injury or any other disease. For example, neuromodulation systems may be used for the treatment of autonomic dysfunction after SCI (or other symptoms occurring in other diseases). The programable device 100 and corresponding methods can improve the use of a neurostimulation device 200 for a subject with at least one impaired body function.

The disclosed embodiments can use the neuromodulation system 10 for treating a subject, with the features described herein. In some embodiments, the neuromodulation system 10 can be used to treating a subject with at least one abnormal body function or disease. As described herein, abnormal body function may be or may comprise any type of pathophysiological condition.

The system 10 can perform a treatment or modulation of at least one of a condition, function, or dysfunction of the subject's gastrointestinal system, cardiovascular system, sensory system, urinary system, respiratory system, reproductive system, thermoregulation system and/or locomotor system. In particular, the specific use of the neuromodulation system 10 for treating a subject may enable that the neuromodulation provided by the neuromodulation system 10 is specifically adapted to at least one of a subject's needs, impairment, rehabilitation status, or environment. This may enable advancing progress in rehabilitation and thus a most efficient and fast rehabilitation process.

In general, the gastrointestinal system of the subject may comprise the upper gastrointestinal tract and/or the lower gastrointestinal tract. In particular, the gastrointestinal system may comprise the mouth, esophagus, stomach and/or intestine of the subject. In particular, the intestine may comprise the small intestine (duodenum, jejunum, ileum) and/or the large intestine (cecum, appendix, ascending colon, right colic flexure, transverse colon, left colic flexure, descending colon, sigmoid colon, rectum and/or anus). In general, sphincters of the respective organs may be included in the gastrointestinal system. Further, the gastrointestinal system of the subject may comprise the liver, gallbladder, pancreas, pancreatic duct and/or immune system of the gastrointestinal system of the subject.

The reproductive system of the subject may comprise testes, scrotum, prostate, penis, urethra, vas deferens, Cowper's gland, vulva, ovaries, breasts and/or cervix of the subject.

The urinary system of the subject may comprise kidney(s), renal pelvis, ureter, urinary bladder, urethra and/or adrenal gland(s) of the subject.

The cardiovascular system of the subject may comprise arteries, capillaries, veins, portal veins, the heart and/or coronary vessels.

The respiratory system of the subject may comprise nose, nasal cavities, sinuses, pharynx, larynx (at least partially), trachea, lung, bronchi, bronchioles and/or alveoli. The sensory system of the subject may comprise sensory neurons, sensory receptor cells, neural pathways and/or parts of the brain involved in sensory perception.

The locomotor system of the subject may comprise the skeleton of the subject and/or at least one muscle, nerve, bone, cartilage and/or tendon. In particular, the locomotor system may comprise upper and lower body parts, including arms, legs, trunk, abdomen, neck, head, etc.

The thermoregulation system of the subject may comprise sweat glands, vascularization (in particular skin vascularization), adipose tissue (in particular white adipose tissue and/or brown adipose tissue), muscles, central nervous system (e.g., Lissauer's tract, spinal cord, brain, e.g., hypothalamus, thalamus) and/or thermoreceptors.

The at least one condition, function, or dysfunction may comprise at least one of acetylcholine dysregulation, acidity of stomach, autonomic dysreflexia, back pain, sphincter function, blood volume dysregulation, breathing deficiency, bronchial tree obstruction, cardiac activity, cholesterol imbalance, motor deficits (e.g., motor deficits being or including a deficit of the motor function of at least one of the upper extremities, lower extremities, trunk, abdomen, neck, or head), mucosa renewal (e.g., mucosa renewal of the intestine, colon and/or stomach), immune system deficit, immobility of the stomach and/or intestine and/or colon, lipogenesis/lipolysis imbalance, lower limb paralysis and/or upper limb paralysis (bilateral and/or unilateral), detrusor muscle, bronchial deficiency, alveolar deficiency, mean venous pressure, mean arterial pressure, mucous layer of stomach, erectile dysfunction, neck stabilization, odor, hypotension, orthostatic hypotension, hypertension, salivary glands function, supraspinal cardiovascular regulation, swallowing, taste, thermoregulation, trunk stabilization, vision, cutaneous vascularization, sweat glands, lipid metabolism and/or glycogen metabolism. The specific use of the neuromodulation system 10 for treating at least one of these conditions and/or functions and/or dysfunctions may enable that physiological deficits of an impaired subject are at least partially restored. This enables the user's 50 performance and/or quality of life to be enhanced.

As used herein, a sphincter may be or may include any autonomous or voluntary regulated sphincter. Without limitation, the term "sphincter" may refer to the upper or lower sphincter of the esophagus, pyloric, ileocecal, urethral (e.g., lower urinary or upper urinary sphincter), or internal or external anal sphincter. Sphincter dysfunction may be or may comprise dysfunction of such a sphincter.

The at least one abnormal body function or disease may be or may comprise myasthenia, dystonia, epilepsy, muscular hyperfatigability, ulcers, Helicobacter Pylori infection, inflammatory bowel disease, Crohn's disease, ulcerative colitis, irritable bowel syndrome, spinal cord injury, incontinence (e.g., incontinence being or including complete fecal or urinary incontinence), hypertension, hypotension, sudden episodic high blood pressure, dyspnea, chronic obstructive pulmonary disease, cardiac failure, cardiac dysrhythmia, bradycardia, tachycardia, cardiac arrest, impairment of genital plexus (e.g. after prostate removal), HIV infection, herniated disk, tumor, obesity, gastroesophageal reflux disease, lower esophageal sphincter failure, achalasia, amyotrophic lateral sclerosis, trauma, stroke, damaged dorsal root, pernicious anemia, vitamin D deficiency, multiple sclerosis, diabetes mellitus, autoimmune disease, Sjogren syndrome, Mikulicz syndrome, loss of sympathetic control, dysphagia, overreactive bladder syndrome, hyperhomocysteinemia, neurodegenerative disease (e.g., neurodegenerative disease being or including Alzheimer's disease or Parkinson's disease), essential tremor, schizophrenia, osteoporosis and/or hyperthyroidism.

Consistent with disclosed embodiments, the specific use of the neuromodulation system 10 for treating a subject at least one of these diseases or abnormal functions may enable that the symptoms of these abnormal functions or diseases to be at least partially cured (or symptoms may be relieved during stimulation). In particular, the specific use of the neuromodulation system 10 may enhance the user's 50 performance or quality of life.

Consistent with disclosed embodiments, treating a user 50 may be or may comprise treating at least one target. A target can be or include a muscular cholinergic synapse, stomach, sympathetic and/or parasympathetic nervous system, heart, cardiac muscle, spinal cord, lower urinary sphincter, autonomous and/or voluntary regulated sphincters, Renin Angiotensin system, kidney, lower bowel sphincter, diaphragm, intercostal muscle, bronchia, smooth muscles of the pulmonary system, solitary tract, sinoatrial/atrioventricular nodes, pancreas, genital plexus, dorsal root to muscle (e.g. pharynx), colon, vasovagal reflex, cajal cell, muscle, smooth muscle, nerve, concerned nerve or group of nerves, concerned plexus (including but not limited to genital, Auerbach, Meissner, cardiac, pulmonary, cervical, lumbar, sacral and/or celiac plexus), liver, adrenal gland, lower esophageal sphincter, alveoli, pulmonary circulation, associated cortex and/or regulatory center, salivary gland and/or detrusor muscle. Accordingly, by treating specific target areas, a high degree of neuromodulation specificity may be achieved.

In some embodiments, such target areas could be treated directly and/or indirectly. For example, a target area may be the actual region of dysfunction (direct treatment) and/or a target area could be a region distal to the actual region of dysfunction but that will still modulate the dysfunction (indirect treatment). A variety of conditions could be modulated by focusing on these target areas. For example, a target area of intercostal muscles may include innermost, internal and/or external intercostal muscles. Similarly, a target area of the respiratory tract may include respiratory system and/or associated blood circulation and/or smooth muscles. A target area of the spinal cord may include dorsal roots, afferent and/or efferent nerves, and/or ganglions.

The use of the neuromodulation system 10 according to the present invention may involve the use of the neuromodulation system 10 throughout rehabilitation of the user 50 and/or during at least one stage of rehabilitation. In particular, the stage of rehabilitation may be or may comprise at least one physical activity such as stabilization in bed, mobilization in bed, mobilization at bedside, verticalization, blood pressure lowering, blood pressure rehabilitation, blood pressure stabilization, cardiac rehabilitation, cardiac stabilization, standing, verticalization, stepping, balance training, locomotor training, fluent locomotion, spinal shock, bowel voidance, bladder voidance, sexual function and/or daily life activities. The stage of rehabilitation may be pre-surgery, post-surgery, pre-implantation, post-implantation, post-setting of neuromodulation system, spinal shock and/or daily life activities. This may enable best possible treatment during each stage and/or phase of rehabilitation.

It is generally possible that the use of the neuromodulation system 10 is specifically adapted to the subject's 50 individual needs and/or preferences and/or rehabilitation stage. It is further generally possible, that the use of the neuromodulation system 50 is altered throughout rehabilitation.

Further, the use of the neuromodulation system 10 may comprise or may be combined with the use of an assistive device, wherein the assistive device may be or may comprise at least one of a breathing device, pulling handles, cables from ceiling, aquatic equipment (noodles, floating vests, boards, etc.), a treadmill, an exoskeleton, an orthosis, a robot, a bike, a breather, crutches, a cuff, a cuff for functional electrical stimulation, a smartwatch, a motorized mattress, non-invasive ventilation, a standing frame, a rollator, a body weight support system, elastic bands, weights, a slide board, a step machine, a trike, a walker, a robot-physiotherapist, balls and/or half-balls. In particular, the use of an assistive device together with a neuromodulation system and/or as part of a neuromodulation system enables efficient progress in rehabilitation.

The neuromodulator 200 may be or may comprise an electrical neurostimulation system and/or mechanical neurostimulation system and/or pharmacological neurostimulation system. It may be possible that the pharmacological neurostimulation system comprises a drug pump. Alternatively, the neurostimulation system may be or may comprise an optical stimulation system, an ultrasound stimulation system and/or a magnetic field stimulation system.

In some embodiments, an electrical stimulation system may be or may comprise a pulse generating system. In particular, the pulse generating system may be an implantable pulse generating system (IPG). The electrical stimulation system may alternatively and/or additionally comprise an electrode array comprising multiple electrodes and/or a lead.

In some embodiments, the neuromodulator 200 may be or may comprise at least one of a transcutaneous electrical stimulation system (TENS), epidural electrical stimulation system (EES), functional electrical stimulation system (FES), intramuscular stimulation system (IMS), dorsal root ganglion stimulation system, subdural stimulation system, cardiac stimulation system, optogenetics, optotrodes, patch-clamp, intra-cellular electrodes and/or extra-cellular electrodes. It may be generally possible that the neuromodulation system is a central nervous system (CNS) stimulation system and/or peripheral nervous system stimulation (PNS) system.

In some embodiments, for the use of the neuromodulator 200 for stimulation of the CNS may comprise that neurostimulation may be applied to any area of the brain and/or spinal cord (including but not limited to spinal nerves, ganglions, nerve roots) and/or nervous plexus and/or glands related to the nervous system (e.g., medulla). In particular, electrical neurostimulation may be applied to at least one of T6 - T9, T6 or higher, T2-L1, L1-L2, S2-S4, T10-L1, T10-L2 and S2-S4, C3-C5, T1-T5, T5-T9, L5-S5, L2-S1, L1-S1, T7-T12, T5-T8, L2-S2, T1-T6, T1-T5, T6-T9, C1-C3, above C1, C1-C4, C1-C4 and above, N.X, N. IX, C1-C5, T7, hypothalamus, medulla, T7-L1, C1-S1, L3-S4.

Epidural Electrical Stimulation (EES) is known to restore motor control in animal and human models and has more particularly been shown to restore locomotion after spinal cord injury by artificially activating the neural networks responsible for locomotion below the spinal cord lesion. EES does not directly stimulate motor-neurons but the afferent sensory neurons prior to entering into the spinal cord. In this way, the spinal networks responsible for locomotion are recruited indirectly via those afferents, restoring globally the locomotion movement by activating the required muscle synergies.

A neuromodulation system 10 consistent with disclosed embodiments may be an open-loop system and/or closed-loop system and/or pseudo-open-loop system. In some embodiments, an open-loop system may be operated manually, with a simple design. Thus, open loop systems may be stable and economical compared to closed loop systems. In some embodiments, a closed-loop system may be accurate even in the presence of non-linearities. The sensitivity of the system may be made small to make the system more stable. The closed loop system may be less affected by noise.

In some embodiments, the closed-loop system may comprise at least one sensor 300. This enables that the patient's status and/or performance may be assessed and/or provided to the neuromodulation system. This may further enable that the neuromodulations performance is specifically adapted to the patient's needs. The sensor 300 may be at least one of an accelerometer, pressure sensor, motion capture sensor, force plate, rehabilitation device, electrode, electrode lead, thermometer (e.g. nose thermometer, skin thermometer, implanted thermometer), infrared camera, doppler ultrasound, electrocardiogram, GPS, smartphone, smartwatch, action-camcorder, video processing, virtual reality module, magnetic resonance imaging, plethysmography sensor, pulse oximetry sensor, pulse pressure sensors, inertial measurement unit (IMU), goniometer, insole, electroencephalography (EEG), skin resistance sensor, infrared sensor, spirometer (with or without dilution), stethoscope, tensiometer, intestinal motility sensor, bowel sensor and/or pH sensor.

The use of the neurostimulation system 10 may comprise and/or may be combined with the use of a feedback system, wherein the feedback system is an audio and/or visual and/or sensory-motoric feedback system. The feedback system can provide the subject with feedback information (e.g., concerning the neuromodulation system's performance and/or the patient's performance and/or patient's body reaction(s)). In this manner, the feedback system can support or enhance rehabilitation. In some embodiments, the feedback system can be part of the closed-loop system. In particular, the audio feedback system may include, for example, headphones, speakers, providing sound or voice signals. The visual feedback system may include, for example, floor projection, goggles, LEDs and/or lights, and/or a screen for providing visual signals.

The sensory-motoric feedback system may provide and/or comprise signals such as haptic signals, pain, paresthesia, signals provided by a person, sensory pathways stimulation, temperature signals and/or changes, touch, vibration etc. The feedback system may be part of the closed-loop system and/or vice versa.

The at least one sensor 300 may provide quality and/or performance metrics of the use of the neuromodulation system 10. The quality and/or performance metrics may be or may comprise sensitivity scores, motor scores, quality of movement scores, performance and/or activity, participation, reaction times, clinical assessment scores, autonomous system scores. In particular, the quality and/or performance scores may be or may comprise autonomous muscular reflexes and/or stretch reflexes, fluidity, blood O₂ saturation, blood speed, distance to path ratio, cardiac parameters, gait parameters, heart rate, force, speed, distance, stress level, muscle strength, range of motion, pulse pressure wave, QoL questionnaires, quantity and timing of urine, respiratory rate, respiratory volume, or stress markers (e.g., stress markers in body fluids).

In some embodiments, the neuromodulation system 10 may provide tonic and/or burst and/or high-frequency neuromodulation. Tonic neuromodulation may be applied with 1 to 125 Hz. Burst neuromodulation may be applied by applying pairs, triplets, quadruplets, quintuplets of pulse at burst frequency of at least 200 Hz, preferably 300 to 750 Hz, at an interburst frequency between 1 and 125 Hz, preferably 10 to 60 Hz. High frequency neuromodulation may be applied with 200 Hz to 10 kHz.

In some embodiments, the neuromodulation system 10 may provide frequency neuromodulation on top of a carrying frequency. In particular, the carrying frequency may be applied with 1 to 10 kHz, preferably 5 to 10 kHz.

In some embodiments, indoor and/or outdoor use of the neuromodulation system 10 may be possible. In some instances, the use of the system may be possible during activities performed by the subject. For example, this may enable use of the neuromodulation system during daily life activities and/or sports (e.g., walking, running, cycling, hiking, horse riding, skiing, swimming, sailing, jumping, ball sports, dancing), etc.

Indoor use may comprise use in least one of clinics, gym, physiotherapy room, occupational room, aquatic room, sport activity room, recreation facility, restaurant, cinema, bathroom, bedroom, kitchen, living room, etc.

Outdoor use may comprise use on streets (including gravel, concrete pavement, cobble stone, rocky hiking trails, etc.), grass, shallow water, sand and/or snow.

It is generally possible that the neuromodulation system 10 may be used and/or operated by at least one robot and/or a person. In particular, the robot may be an assistive robot for healthcare and/or a humanoid robot. In particular, the person may be a family member, a friend, a therapist, a physiotherapist, a nurse, an electrophysiologist, a non-professional user, a professional user, an occupational therapist, a surgeon, a technician (clinical/ FCE), and/or a (treating) physician of the subject and/or the subject himself. Further, the neuromodulation system may be used by a virtual assistant. Additionally, and/or alternatively, the neuromodulation system may be operated locally or remotely.

Consistent with disclosed embodiments, the use of the neuromodulation system 10 may comprise the use of a network and/or device and/or platform. In particular, the use of the neuromodulation system may comprise the use of at least one of a cloud, game console, internet, local database, local network, medical and/or rehabilitation device, smartphone, smartwatch and/or social media.

In some embodiments, the use of the neuromodulation system 10 may be performed to perform a physical activity such as a task including but not limited to balancing, bending over, coughing, walking, climbing, climbing stairs, swimming and/or performing other tasks under water, sitting, sitting on ground, sit-to-stand, grasping, squatting, standing, swallowing, coughing, , bowel voidance, bladder voidance, sexual function, erecting, inclined waking, neck training, posture training, normal neck posture, normal gait, normal intestinal movement during digestion, normal trunk posture, (normal( use of at least one affected limb, (normal) use of lower limbs, prevention of muscle weakness, regulation of stomach acidity and mucosal layer, respiration, transferring, voluntary regulated sphincter action, zero-G-training and/or performing above-mentioned tasks in zero-G.

The use of the neuromodulation system 10 may be performed to evoke responses such as but not limited to cardiac response, hormonal response, motor response, neurotransmitter response, sensory response, smooth muscle contraction, smooth muscle dilatation, general somatic afferent response (perception of touch, pain and/or temperature and/or others), general somatic efferent response (voluntary motor innervation and/or others), general visceral efferent response (motor innervation to smooth muscles and/or heart muscle and/or glands), special afferent response (visceral: smell, taste, somatic: vision, hearing, balance), respiration, cough and/or erection.

### Gastrointestinal system:

In some embodiments, a neuromodulation system 10 consistent with disclosed embodiments can be used to treat and/or modulate of at least one condition and/or function and/or dysfunction of the subject's gastrointestinal system. The at least one condition and/or function and/or dysfunction can be or include bowel sphincter function. In various embodiments, the at least one condition and/or function could be or include at least one of acidity of stomach, cholesterol imbalance, lipogenesis/lipolysis imbalance, mucosa renewal, intestinal immobility, lower esophageal sphincter function, salivary glands function, mucous layer of stomach function, swallowing, taste, immune system deficit, lipid metabolism and/or glycogen metabolism. In some embodiments, the at least one abnormal body function and/or disease can be incontinence.

In various embodiments, the abnormal body and/or disease could be or could comprise ulcers, Helicobacter Pylori infection, inflammatory bowel disease, Crohn's disease, ulcerative colitis, irritable bowel syndrome, tumor, gastroesophageal reflux disease, lower esophageal sphincter failure, achalasia, trauma, spinal cord injury, stroke, tumor, pernicious anemia, vitamin D deficiency, Sjogren syndrome, Mikulicz syndrome, dysphagia, diabetes and/or obesity.

Consistent with disclosed embodiments, treating the subject 50 can include treating the subject's lower bowel sphincter. In various embodiments, treating the subject comprises treating a voluntary regulated sphincter of the subject. In various embodiments, treating the subject could be or could comprise treating at least one of stomach, autonomous regulated sphincter, pancreas, dorsal root to muscle (e.g., pharynx), circulatory cholesterol uptake, colon, vago-vagal reflex, cajal cell, smooth muscle, nerve, Auerbach plexus, Meissner plexus, lower esophageal sphincter, liver, pancreas and/or salivary gland.

In some embodiments, the neuromodulation system 10 can provide treatment to evoke or assist physical activities such as voluntary regulated sphincter action. In various embodiments, the neuromodulation system 10 can provide treatment to evoke or assist other physical activities such as normal intestinal movement during digestion, regulation of stomach acidity and/or mucosal layer, eating, swallowing and/or drinking. Furthermore, the system 10 can be used during activities performed by the subject.

In some embodiments, the neuromodulation system 10 can be a neurostimulation system, wherein the neurostimulation system can include an electrical neurostimulation system. However, alternatively and/or additionally, a mechanical neurostimulation system and/or pharmacological neurostimulation system could be generally possible. In this embodiment, the electrical neurostimulation system can be an intramuscular stimulation system.

In an alternative embodiment, the electrical neurostimulation system could be or could include at least one of a transcutaneous electrical stimulation system, epidural electrical stimulation system, functional electrical stimulation system, dorsal root ganglion stimulation system, subdural stimulation system, optogenetics, optotrodes, patch-clamp, intra-cellular electrodes and/or extra-cellular electrodes.

In some instances, for treating ulcers, Helicobacter pylori infection, Crohn's disease, irritable bowel syndrome and/or pernicious anemia, an electrical stimulation system (e.g., epidural and/or subdural stimulation system) could be used to provide stimulation at least partially to the level of T6-T9 and/or L1-S1 of the subject.

In some instances, for treating incontinence (e.g., after spinal cord injury), an electrical stimulation system (e.g., epidural and/or subdural stimulation system) could be used to provide stimulation at least partially to the level of T10-L2 and/or S2 and/or S4 of the subject.

In some instances, for treating diabetes mellitus, an electrical stimulation system (e.g., epidural and/or subdural stimulation system) could be used to provide stimulation at least partially to the level of T7-T12 and/or T5-T8 of the subject.

In some instances, for treating gastroesophageal reflux disease and/or achalasia, an electrical stimulation system (e.g., epidural and/or subdural stimulation system) could be used to provide stimulation at least partially to N.X.

In some instances, for treating pernicious anemia, vitamin D deficiency, diabetes mellitus, Sjogren syndrome, and/or Mikulicz syndrome the electrical stimulation system (e.g., epidural and/or subdural stimulation system) could be used to provide stimulation at least partially to the level of C1-C4 of the subject.

In some instances, for treating dysphagia (e.g., after spinal cord injury), an electrical stimulation system (e.g., epidural and/or subdural stimulation system) could be used to provide stimulation at least partially to N.X., N.IX and/or the level of C1-C5 of the subject.

In some instances, for use of the neuromodulation system 10 could be performed to evoke responses such as defecation.

Consistent with disclosed embodiments, the neuromodulation system 10 can be an open-loop system, a closed-loop system, or a pseudo-open-loop system. In some embodiments, a sensor 300 of a closed-loop neuromodulation device could be or could comprise at least one of intestinal motility sensor, bowel sensor, pH sensor and/or another sensor.

Consistent with disclosed embodiments, the neuromodulation system 10 can be used post implantation of the neuromodulation system. In various embodiments, the neuromodulation system can be used throughout rehabilitation of the subject and/or during at least one stage of stabilization in bed, mobilization in bed, mobilization at bedside, verticalization, surgery, post-surgery, pre-implantation, post-implantation, spinal shock and/or daily life activities.

Consistent with disclosed embodiments, the neuromodulation system 10 can be used indoors. For example, the indoor use can include use in clinics. In various embodiments, the indoor use could include the use in at least one of gym, physiotherapy room, occupational room, aquatic room, sport activity room, recreation facility, restaurant, cinema, bathroom, bedroom, kitchen, living room, etc.

Consistent with disclosed embodiments, the neuromodulation system 10 could be used outdoors. In some embodiments, the system can be used during activities performed by the subject. In some embodiments, the neuromodulation system 10 can be used or operated by at least one person.

Consistent with disclosed embodiments, the neuromodulation system 10 can be used by the subject 50. In some embodiments, any other person, such as a family member, a friend, a therapist, a physiotherapist, a nurse, an electrophysiologist, a non-professional user, a professional user, an occupational therapist, a surgeon, a technician (clinical/ FCE) and/or a (treating) physician of the subject could use the system. In some embodiments, the neuromodulation system can be operated by a robot.

### Reproductive system

Consistent with disclosed embodiments, the use of a neuromodulation system 10 for treating a subject with at least one abnormal body function and/or disease comprises the treatment and/or modulation of at least one condition and/or function and/or dysfunction of the subject's reproductive system. In some embodiments, the at least one condition and/or function and/or dysfunction can be erectile dysfunction. In some embodiments, the at least one abnormal body function and/or disease can be impairment of genital plexus after prostate removal.

In some embodiments, the at least one abnormal body function and/or disease can be or include at least one of stroke, trauma, spinal cord injury, tumor, diabetes, Parkinson's disease, Alzheimer's disease and/or neurodegenerative disease. Consistent with disclosed embodiments, treating a subject can include treating at least one of the genital plexus, smooth muscle, or nerves of the subject. In some embodiments, the use of the neuromodulation system 10 can be performed throughout rehabilitation of the subject. In various embodiments, the use of the neuromodulation system 10 can be performed during at least one stage of rehabilitation.

Consistent with disclosed embodiments, the stage of rehabilitation could be or could include at least one of stabilization in bed, mobilization in bed, mobilization at bedside, verticalization, blood pressure lowering, blood pressure rehabilitation, cardiac rehabilitation, standing, stepping, balance training, locomotor training, fluent locomotion, pre-surgery, post-surgery, post-implantation, post-setting of neuromodulation system, spinal shock and/or daily life activities.

Consistent with disclosed embodiments, the neuromodulation system can be a neurostimulation system, wherein the neurostimulation system can include an electrical neurostimulation system. However, in various embodiments, the neurostimulation system can be or include a mechanical neurostimulation system and/or pharmacological neurostimulation system.

Consistent with disclosed embodiments, the electrical neurostimulation system 10 can be an epidural electrical stimulation system. In this embodiment, the epidural electrical stimulation system can be used to provide stimulation at least partially to level L5-S5 of the subject.

In some embodiments, the stimulation system 10 could be alternatively and/or additionally at least one of a transcutaneous electrical stimulation system, functional electrical stimulation system, intramuscular stimulation system, dorsal root ganglion stimulation system, subdural stimulation system, optogenetics, optotrodes, patch-clamp, intra-cellular electrodes and/or extra-cellular electrodes.

Consistent with disclosed embodiments, the use of the neuromodulation system 10 is performed to evoke responses such as erection. In various embodiments, any other type of response, such as motor and/or sensory response of the reproductive system could be generally possible.

Consistent with disclosed embodiments, the neuromodulation system 10 may be an open-loop system. In some embodiments, the neuromodulation system may be a closed-loop system and/or pseudo-open-loop system. Consistent with disclosed embodiments, the use of the neuromodulation system can include the use of burst neuromodulation. In some embodiments, the burst neuromodulation can be applied by applying pairs, triplets, quadruplets, quintuplets of pulse at burst frequency of at least 200 Hz, preferably 300 to 750 Hz, at an interburst frequency between 1 and 125 Hz, preferably between 10 and 60 Hz. In various embodiments, the use of tonic and/or high-frequency neuromodulation, in particular neurostimulation could be generally possible.

Consistent with disclosed embodiments, the system 10 could provide frequency neuromodulation on top of a carrying frequency. For example, the carrying frequency could be applied with 1 to 10 kHz, preferably 5 to 10 kHz.

In some embodiments, the use of the neuromodulation system 10 could include the use of an assistive device. In various embodiments, the neuromodulation system could be used without use of an assistive device.

### Urinary system

Consistent with disclosed embodiments, the neuromodulation system 10 can be used for the treatment and/or modulation of at least one condition and/or function and/or dysfunction of a subject's urinary system. In some embodiments, the at least one condition and/or function and/or dysfunction could be bladder sphincter function. For example, the neuromodulation system 10 could be used for the treatment and/or modulation of bladder sphincter dysfunction. In various embodiments, the at least one condition and/or function and/or dysfunction can include at least one of blood volume, mean arterial pressure, vesical sphincter, or other functions, conditions, or dysfunctions suitable for treatment according to disclosed embodiments.

Consistent with disclosed embodiments, the at least one abnormal body function and/or disease can be incontinence. In various embodiments, the at least one abnormal body function and/or disease could be or could comprise hypertension, hypotension, sudden episodic high blood pressure, trauma, spinal cord injury, stroke, tumor, damaged dorsal root, Parkinson's disease, Alzheimer's disease, neurodegenerative disease, overreactive bladder syndrome and/or others.

Consistent with disclosed embodiments, treating the subject can include treating at least one muscle of the subject. In some instances, treating the subject can include treating the lower urinary sphincter of the subject. In some embodiments, the neuromodulation system 10 can be used to treat at least one of sympathetic and/or parasympathetic nervous system, autonomous and/or voluntary regulated sphincters, smooth muscles, nerves, kidney, detrusor, kidney and/or anatomical structures suitable for treatment according to the disclosed embodiments.

Consistent with disclosed embodiments, the use of the neuromodulation system 10 can include the use of the neuromodulation system 10 throughout rehabilitation of the subject. In some embodiments, the use of the neuromodulation system 10 can include the use of a neuromodulation system 10 during at least one stage of rehabilitation.

Consistent with disclosed embodiments, the stage of rehabilitation could be or could comprise at least one of stabilization in bed, mobilization in bed, mobilization at bedside, verticalization, blood pressure lowering, blood pressure rehabilitation, cardiac rehabilitation, standing, stepping, balance training, locomotor training, fluent locomotion, pre-surgery, post-surgery, pre-implantation, post-implantation, post-setting of neuromodulation system, spinal shock and/or daily life activities.

In some embodiments, the neuromodulation system 10 can be a neurostimulation system 10. The neurostimulation system 10 can be or include at least one of an electrical neurostimulation system, a mechanical neurostimulation system, or a pharmacological neurostimulation system. In some embodiments, the electrical neurostimulation system can be an epidural electrical stimulation system.

Consistent with disclosed embodiments, the epidural electrical stimulation system can be used to provide stimulation at least partially to level L1-L2 and/or S2-S4 of the subject. Alternatively, the epidural electrical stimulation system can be used to provide stimulation at least partially to level L3-L4 of the subject.

Consistent with disclosed embodiments, the neurostimulation system 10 could be or include at least one of a transcutaneous electrical stimulation system, functional electrical stimulation system, intramuscular stimulation system, dorsal root ganglion stimulation system, subdural stimulation system (also for the treatment of overreactive bladder syndrome), optogenetic stimulation system, optotrode stimulation system, patch-clamp, or intra-cellular electrodes or extra-cellular electrode stimulation system.

In some embodiments, for treating hypotension and/or hypertension, the electrical stimulation system (e.g., epidural and/or subdural stimulation system) can be used to provide stimulation at least partially to level T10-L1 of the subject.

Consistent with disclosed embodiments, the neuromodulation system can be used indoors or outdoors. In some embodiments, the use of the system can be possible during activities performed by the subject.

Consistent with disclosed embodiments, the neuromodulation system 10 can be used to provide tonic neuromodulation, in particular tonic neurostimulation. In some instances, tonic neuromodulation could be applied with 1 to 125 Hz. In some embodiments, the neuromodulation system 10 can provide frequency neuromodulation on top of a carrying frequency. In some embodiments, the carrying frequency can be between 1 to 10 kHz, preferably between 5 to 10 kHz. In some embodiments, the neuromodulation system 10 can provide burst and/or high-frequency neuromodulation.

Consistent with disclosed embodiments, the neuromodulation system 10 can be a closed-loop system. In some embodiments, the neuromodulation system can be an open-loop system or a pseudo-open-loop system. Consistent with disclosed embodiments, the closed-loop neuromodulation system can include a sensor 300. The sensor 300 of such a closed-loop neuromodulation system can be or include at least one of a tensiometer and/or electrocardiogram and/or any other type of sensor.

Consistent with disclosed embodiments, the neuromodulation system 10 can be used or operated by at least one person. The person can be a family member, a friend, a therapist, a physiotherapist, a nurse, an electrophysiologist, a non-professional user, a professional user, an occupational therapist, a surgeon, a technician (clinical/ FCE), and/or a (treating) physician of the subject. In some embodiments, the neuromodulation system can be operated by a robot.

Consistent with disclosed embodiments, the neuromodulation system 10 can be used together with an assistive device. In various embodiments, an assistive device need not be used together with the neuromodulation system 10.

Consistent with disclosed embodiments, the neuromodulation system 10 can be configured to provide neuromodulation stimulation for evoking a response of the sphincter (e.g., a contraction or relaxation).

### Cardiovascular system

In some embodiments, a neuromodulation system 10 consistent with disclosed embodiments can be used to treat and/or modulate of at least one condition and/or function and/or dysfunction of a subject's cardiovascular system. The at least one condition and/or function and/or dysfunction can be or include mean venous pressure or mean arterial pressure. In some embodiments, the abnormal body function can be or include hypertonia. In various embodiments, the at least one condition and/or function and/or dysfunction could include at least one of cardiac activity, supraspinal cardiovascular regulation, autonomic dysreflexia, orthostatic hypotension and/or mean venous pressure or mean arterial pressure.

In some embodiments, the abnormal body function and/or disease can be or include dystonia, spinal cord injury, hypotension, sudden episodic high blood pressure, supraspinal cardiovascular regulation, cardiac failure, cardiac dysrhythmia, bradycardia, tachycardia, cardiac arrest, tumor, trauma and/or stroke.

Consistent with disclosed embodiments, treating the subject can include treating nerves of the subject. In various embodiments, treating the subject can include treating the spinal cord of the subject.

Consistent with disclosed embodiments, the treatment of a subject can be performed directly and/or indirectly. In various embodiments, treating the subject can include treating at least one of heart, cardiac muscle, muscle, smooth muscles, nerves, Renin Angiotensin system, kidney, adrenal gland and/or sinoatrial/atrioventricular nodes.

Consistent with disclosed embodiments, the neuromodulation system 10 can be a neurostimulation system 10. The neurostimulation system 10 can be or include at least one of an electrical neurostimulation system, mechanical neurostimulation system, or pharmacological neurostimulation system.

Consistent with disclosed embodiments, the neurostimulation system 10 can be an epidural electrical stimulation system. In some embodiments, the epidural electrical stimulation system can be used to provide stimulation at least partially to the level of T10-L1 of the subject. In some embodiments, the stimulation system could be alternatively and/or additionally at least one of a transcutaneous electrical stimulation system, functional electrical stimulation system, intramuscular stimulation system, dorsal root ganglion stimulation system, subdural stimulation system, cardiac stimulation system, optogenetics, optotrodes, patch-clamp, intra-cellular electrodes and/or extra-cellular electrodes.

In some embodiments, an electrical stimulation system 10 (e.g., epidural and/or subdural stimulation system) can be used to provide stimulation at least partially to the level of T6 or higher and/or T1-T6 of the subject.

In some embodiments, for treating cardiac failure and/or spinal cord injury, an electrical stimulation system (e.g., epidural and/or subdural stimulation system) could be used to provide stimulation at least partially to the level of T1-T5 of the subject.

In some embodiments, for treating hypertonia and/or spinal cord injury (e.g., when autonomic dysreflexia occurs) the electrical stimulation system (e.g., epidural and/or subdural stimulation system) could be used to provide stimulation at least partially at to level of C1-C4 of the subject and above.

In various embodiments, the neurostimulation system 10 can be a dorsal root ganglion stimulation system and/or subdural stimulation system or cardiac stimulation system.

In certain embodiments, the neuromodulation system 10 can be used post-implantation in the subject. For example, an epidural electrical stimulation system may be used post-implantation in the subject.

Consistent with disclosed embodiments, the neuromodulation system 10 can be used throughout rehabilitation of the subject and/or during at least one stage of rehabilitation. In particular, the neuromodulation system 10 can be used at a rehabilitation stage including at least one of stabilization in bed, mobilization in bed, mobilization at bedside, verticalization, blood pressure lowering, blood pressure rehabilitation, blood pressure stabilization, cardiac rehabilitation, cardiac stabilization standing, stepping, balance training, locomotor training, fluent locomotion, pre-surgery, post-surgery, pre-implantation, post-setting of neuromodulation system 10, spinal shock and/or daily life activities.

Consistent with disclosed embodiments, the neuromodulation system 10 can be used indoors or outdoors. In some embodiments, the neuromodulation system 10 can be used during activities performed by the subject.

Consistent with disclosed embodiments, the neuromodulation system 10 can perform neuromodulation to modulate smooth muscle contraction (response). In various embodiments, the neuromodulation system 10 can perform neuromodulation to evoke responses such as but not limited to cardiac response, hormonal response, neurotransmitter response, sensory response and/or smooth muscle dilatation.

Consistent with disclosed embodiments, the use of the neuromodulation system 10 can include the use of tonic neuromodulation, in particular, tonic neurostimulation. In some embodiments, the tonic neuromodulation may be applied with 1 to 125 Hz. In some embodiments, the neuromodulation system 10 could provide frequency neuromodulation on top of a carrying frequency. For example, the carrying frequency could be applied with 1 to 10 kHz, preferably 5 to 10 kHz. In various embodiments, the neuromodulation system 10 could provide burst or high-frequency neuromodulation (e.g., neurostimulation).

Consistent with disclosed embodiments, the neuromodulation system 10 can be an open-loop system. In some embodiments, the neuromodulation system 10 can be a closed-loop system or pseudo-open-loop system. In some embodiments, a sensor 300 of such a closed-loop neuromodulation system 10 can be or include at least one of a tensiometer, pulse pressure measuring method, electrocardiogram, pulse oximetry, MRI, doppler ultrasound and/or any other type of sensor. The at least one sensor 300 can provide quality and/or performance metrics of the use of the neuromodulation system 10. The quality and/or performance metrics can be or include cardiac parameters. In some embodiments, the neuromodulation system 10 can include at least one sensor 300 which may include a pressure sensor, a rehabilitation device, an electrode, an electrode lead, an electrocardiogram, magnetic resonance imaging, a pulse oximetry sensor, or a pulse pressure sensor.

Consistent with disclosed embodiments, the quality and/or performance metrics could comprise fatigue measures, stress measures (e.g., in body fluids), questionnaires (in particular QoL questionnaires), pulse pressure wave, blood 02 saturation, blood speed, QoL questionnaires, q and/or stress markers (e.g., stress markers in body fluids).

Consistent with disclosed embodiments, the neuromodulation system 10 can provide treatment together with an assistive device. In various embodiments, the neuromodulation system 10 can be used without an assistive device. Such an assistive device can be or include at least one of pulling handles, cables from ceiling, aquatic equipment, a treadmill, an exoskeleton, an orthosis, a robot, a bike, a breather, crutches, a cuff, a cuff for functional electrical stimulation, a smartwatch, a motorized mattress, non-invasive ventilation, a standing frame, a rollator, a body weight support system, elastic bands, weights, a slide board, a step machine, a trike, a walker, a robot-physiotherapist, balls and/or half-balls.

Consistent with disclosed embodiments the neuromodulation system 10 can be used by a (treating) physician. In various embodiments, the neuromodulation system 10 can be used by another person. Such a person could be family member, a friend, a therapist, a physiotherapist, a nurse, an electrophysiologist, a non-professional user, a professional user, an occupational therapist, a surgeon and/or a technician (clinical/ FCE) of the subject.

Consistent with disclosed embodiments, the use of the neuromodulation system 10 can include the use of a network device. In some embodiments, the use of the neuromodulation system can include the use of a smartphone. In various embodiments, the use of the neuromodulation system could include the use of a network and/or platform and/or alternative network device. In some embodiments, the use of the neuromodulation system can include the use of at least one of a cloud, game console, internet, local database, local network, medical and/or rehabilitation device, smartwatch and/or social media.

Consistent with disclosed embodiments, the use of the neurostimulation system can include the use of a feedback system. The feedback system can be a visual feedback system. In some embodiments, the feedback system can be a screen. In various embodiments, the feedback system can be or can include an audio feedback system and/or sensory-motoric feedback system. In some instances, the feedback system can be or include headphones and/or haptic signals.

### Respiratory system

In some embodiments, a neuromodulation system 10 consistent with disclosed embodiments can be used to treat and/or modulate of at least one condition and/or function and/or dysfunction of a subject's respiratory system. The at least one condition and/or function and/or dysfunction can be or include a breathing deficiency. In some embodiments, at least one abnormal body function can be or include dyspnea. In various embodiments, the at least one condition and/or function and/or dysfunction could include at least one of bronchial tree obstruction and/or bronchial deficiency and/or alveolar deficiency.

In some embodiments, the at least one abnormal body function and/or disease can be or include at least one of chronic obstructive pulmonary disease, tumor, trauma, stroke, damaged dorsal root, spinal cord injury and/or amyotrophic lateral sclerosis (ALS).

Consistent with disclosed embodiments, treating a subject can include treating the intercostal muscles of the subject. In various embodiments, treating the subject can include treating at least one of bronchia, smooth muscles of the pulmonary system, nerve, alveoli, muscles, solitary tract, pulmonary circulation and/or diaphragm.

Consistent with disclosed embodiments, the neuromodulation system 10 can be a neurostimulation system 10. The neurostimulation system 10 can be or include at least one of an electrical neurostimulation system, mechanical neurostimulation system, or pharmacological neurostimulation system.

Consistent with disclosed embodiments, the neurostimulation system 10 can be an intramuscular stimulation system. In various embodiments, the neurostimulation system can be a dorsal root ganglion stimulation system and/or subdural stimulation system and/or transcutaneous electrical stimulation system and/or epidural electrical stimulation system.

In some embodiments, for treating dyspnea and/or spinal cord injury (e.g., for treating dyspnea after spinal cord injury) and/or chronic obstructive pulmonary disease, the electrical stimulation system (e.g., epidural and/or subdural stimulation system) can be used to provide stimulation at least partially to the level of C3-C5 of the subject.

In some embodiments, for treating ALS (e.g., breathing deficiency caused by ALS), the electrical stimulation system (e.g., epidural and/or subdural stimulation system) can be used to provide stimulation at least partially to the level of T1-T6 of the subject.

In some embodiments, the neuromodulation system 10 can be used post-implantation in the subject, in particular post implantation of the intramuscular stimulation system.

In various embodiments, the use of the neuromodulation system 10 can include the use of the neuromodulation system 10 throughout rehabilitation of the subject and/or during at least one stage of stabilization in bed, mobilization in bed, mobilization at bedside, verticalization, stabilization of blood pressure, standing, stepping, balance training, locomotor training, fluent locomotion, pre-surgery, post-surgery, pre-implantation, post-implantation, post-setting of neuromodulation system 10, spinal shock and/or daily life activities.

Consistent with disclosed embodiments, the system 10 can be used indoors. In some embodiments, such indoor use can include use in least one of clinics, gym, physiotherapy room, occupational room, aquatic room, sport activity room, recreation facility, restaurant, cinema, bathroom, bedroom, kitchen, living room, or similar indoor environments. In various embodiments, the neuromodulation system can be used outdoors. Consistent with disclosed embodiments, the neuromodulation system 10 can be used during activities performed by the subject.

Consistent with disclosed embodiments, the neuromodulation system 10 can be used together with an assistive device. In various embodiments, an assistive device need not be used together with the neuromodulation system.

Consistent with disclosed embodiments, the neuromodulation system 10 can be configured to provide tonic neuromodulation (e.g., tonic neurostimulation). In some embodiments, tonic neuromodulation could be applied at frequencies between 1 and 125 Hz.

Consistent with disclosed embodiments, the neuromodulation system 10 can provide frequency neuromodulation on top of a carrying frequency. In particular, the carrying frequency can be applied with 1 to 10 kHz, preferably 5 to 10 kHz. In some embodiments, the neuromodulation system can provide burst and/or high-frequency neuromodulation (e.g., neurostimulation).

Consistent with disclosed embodiments, the neuromodulation system 10 can be a closed-loop system. In some embodiments, the neuromodulation system 10 can be an open-loop system or a pseudo-open-loop system. In some embodiments, the closed-loop system can include one or more sensors 300. The one or more sensors 300 can be or include at least one of a plethysmography sensor, a thermometer (e.g., a nose thermometer), a pressure sensor, a pulse oximetry sensor, an inertial measurement unit (IMU), a goniometer, an insole, an electroencephalography sensor (EEG), an electromyography sensor (EMG), a trans-thoracic impedance sensor, or another suitable sensor.

Consistent with disclosed embodiments, the at least one sensor 300 can provide quality and/or performance metrics of the use of the neuromodulation system 10. In some embodiments, the quality and/or performance metric can be a respiratory rate. In various embodiments, the quality and/or performance metrics can be or include at least one of respiratory volume, respiration rate, blood 02 saturation, or stress markers (e.g., stress markers measured in body fluids).

Consistent with disclosed embodiments, the neuromodulation system 10 can be used or operated by at least one person. In some embodiments, the neuromodulation system 10 can be used by the subject; or by at least one of a family member, a friend, a therapist, a physiotherapist, a nurse, an electrophysiologist, a non-professional user, a professional user, an occupational therapist, a surgeon, a technician (clinical/ FCE), and/or a (treating) physician of the subject.

Consistent with disclosed embodiments, the neuromodulation system 10 can provide neuromodulation configured to evoke a response. The response can be at least one of respiration or a cough.

### Sensory system

Consistent with disclosed embodiments, the neuromodulation system 10 can be used for the treatment and/or modulation of at least one condition and/or function and/or dysfunction of a subject's sensory system. In some embodiments, the at least one condition and/or function and/or dysfunction could be or include at least one of back pain, odor, or taste.

Consistent with disclosed embodiments, the at least one abnormal body function and/or disease can be trauma. In various embodiments, the at least one abnormal body function and/or disease can be or include at least one of a tumor, a herniated disk, Parkinson's disease, Alzheimer's disease, a neurodegenerative disease, a stroke, or spinal cord injury.

Consistent with disclosed embodiments, treating the subject can include treating at least one nerve of the subject. The treatment of the subject can be performed directly and/or indirectly.

Consistent with disclosed embodiments, the neuromodulation system 10 can be a neurostimulation system. The neurostimulation system can be or include at least one of an electrical neurostimulation system, a mechanical neurostimulation system, or a pharmacological neurostimulation system.

Consistent with disclosed embodiments, the neurostimulation system 10 can be an epidural electrical stimulation system. In some embodiments, the epidural electrical stimulation system can be used to provide stimulation at least partially to the level of T2-L1 of the subject. In various embodiments, the epidural electrical stimulation system can be used to provide stimulation at least partially above C1 of the subject.

Consistent with disclosed embodiments, an electrical neurostimulation system can be or include at least one of transcutaneous electrical stimulation system, dorsal root ganglion stimulation system and/or subdural stimulation system.

Consistent with disclosed embodiments, the neuromodulation system 10 can be used post-implantation in a subject. For example, an epidural electrical stimulation system can be used post implantation in the subject. In various embodiments, the neuromodulation system can be used throughout rehabilitation of the subject and/or during at least one of stabilization in bed, mobilization in bed, mobilization at bedside, verticalization, blood pressure lowering, blood pressure rehabilitation, blood pressure stabilization, cardiac rehabilitation, cardiac stabilization, standing, stepping, balance training, locomotor training, fluent locomotion, pre-surgery, post-surgery, pre-implantation, post-setting of neuromodulation system, spinal shock and/or daily life activities.

Consistent with disclosed embodiments, the neuromodulation system 10 can be a closed-loop system. In some embodiments, the neuromodulation system can be an open-loop system or a pseudo-open-loop system. Consistent with disclosed embodiments, the closed-loop neuromodulation system can include a sensor.

Consistent with disclosed embodiments, the neuromodulation system 10 can be configured to use burst neuromodulation (e.g., burst neurostimulation). In some embodiments, the neuromodulation system can be configured to apply burst neuromodulation comprising pairs, triplets, quadruplets, quintuplets of pulses. The pulses can have a burst frequency of at least 200 Hz, preferably 300 to 750 Hz. The pulses can have an interburst frequency of between 1 and 125 Hz, preferably between 10 and 60 Hz.

Consistent with disclosed embodiments, the neuromodulation system 10 can be configured to provide frequency neuromodulation on top of a carrying frequency. In particular, the carrying frequency could be applied at between 1 and 10 kHz, preferably between 5 and 10 kHz. In various embodiments, the neuromodulation system 10 can be configured to use of tonic and/or high-frequency neuromodulation (e.g., neurostimulation).

Consistent with disclosed embodiments, the neuromodulation system 10 can be used indoors or outdoors. In some embodiments, the neuromodulation system can be adapted for use in a clinic, gym, physiotherapy room, occupational room, aquatic room, sport activity room, recreation facility, restaurant, cinema, bathroom, bedroom, kitchen, living room, or other suitable room. In some embodiments, the use of the system can be possible during activities performed by the subject.

Consistent with disclosed embodiments, the neuromodulation system 10 can be used or operated by at least one person. The person can be the subject; or at least one of a family member, a friend, a therapist, a physiotherapist, a nurse, an electrophysiologist, a non-professional user, a professional user, an occupational therapist, a surgeon, a technician (clinical/ FCE), and/or a (treating) physician of the subject.

### Locomotor system

Consistent with disclosed embodiments, the neuromodulation system can be used for the treatment and/or modulation of at least one condition and/or function and/or dysfunction of a subject's locomotor system. In some embodiments, the at least one condition and/or function and/or dysfunction could be or include a gait deficit. In various embodiments, the at least one condition and/or function and/or dysfunction can include at least one of individual limb paralysis, lower limb paralysis, upper limb paralysis, neck stabilization and/or trunk stabilization, or other functions, conditions, or dysfunctions suitable for treatment according to disclosed embodiments.

Consistent with disclosed embodiments, the at least one abnormal body function and/or disease can be spinal cord injury. In various embodiments, the at least one abnormal body function or disease can be or include at least one of epilepsy, muscular hyperfatigability, herniated disk, tumor, amyotrophic lateral sclerosis, multiple sclerosis, trauma, stroke, damaged dorsal root, autoimmune disease, loss of sympathetic control, neurodegenerative disease, Parkinson's disease, essential tremor, Alzheimer's disease and/or osteoporosis.

Consistent with disclosed embodiments, treating the subject can include treating the spinal cord of the subject. In various embodiments, treating a subject can include treating at least one of muscular cholinergic synapse, sympathetic and/or parasympathetic nervous system, spinal cord, dorsal root to muscle, muscle, or smooth muscle and/or nerve.

Consistent with disclosed embodiments, the neuromodulation system 10 can be used during locomotor training of the subject. In various embodiments, the neuromodulation system 10 could be used throughout rehabilitation of the subject and/or during at least one of mobilization in bed, mobilization at bedside, verticalization, stepping, balance training, fluent locomotion, pre-surgery, post-surgery, pre-implantation, post-implantation, post-setting of neuromodulation system 10, spinal shock and/or daily life activities.

Consistent with disclosed embodiments, the neuromodulation system 10 can be used together with an assistive device. For example, use of the neuromodulation system 10 can include the use of an assistive device or be combined with the use of the assistive device. In some embodiments, the assistive device can be an exoskeleton. In various embodiments, the assistive device can be or include at least one of pulling handles, cables from ceiling, aquatic equipment, a treadmill, an orthosis, a robot, a bike, crutches, a cuff, a cuff for functional electrical stimulation, a smartwatch, a motorized mattress, a standing frame, a rollator, a body weight support system, elastic bands, weights, a slide board, a step machine, a trike, a walker, a robot-physiotherapist, balls and/or half-balls.

Consistent with disclosed embodiments, the neuromodulation system 10 can be configured to provide neuromodulation stimulation to evoke or assist a normal gait of a subject. In various embodiments neuromodulation system can be configured to provide neuromodulation stimulation to evoke or assist a task of the subject including at least one of balancing, bending over, walking, climbing, climbing stairs, swimming and/or performing other tasks under water, sitting, sitting on ground, sit-to-stand, grasping, squatting, standing, inclined waking, neck training, posture training, normal neck posture, normal trunk posture, normal use of at least one affected limb, normal use of lower limbs, prevention of muscle weakness, or training with body weight support and/or performing above-mentioned tasks with body weight support.

Consistent with disclosed embodiments, the neuromodulation system can be or include a neurostimulation system 10. The neurostimulation system 10 can be or include at least one of an electrical neurostimulation system, a mechanical neurostimulation system, or a pharmacological neurostimulation system. In some embodiments, an electrical neurostimulation system can be an epidural electrical stimulation system. In various embodiments, the electrical neurostimulation system can be or include at least one of transcutaneous electrical stimulation system, dorsal root ganglion stimulation system, intramuscular stimulation system and/or subdural stimulation system.

Consistent with disclosed embodiments, the electrical stimulation system can be used to provide stimulation at least partially to the level of L2-S1 of the subject. In various embodiments, the electrical stimulation system can be used to provide stimulation at least partially to the level of L2-S2 of the subject.

Consistent with disclosed embodiments, when the neuromodulation system 10 is used for neck stabilization, the electrical stimulation system can be used to provide stimulation at least partially to the level of T2-L1, preferably to the level of T7-L1 of the subject.

Consistent with disclosed embodiments, when the neuromodulation system 10 is used for treating upper and/or lower limb paralysis, the electrical stimulation system can be used to provide stimulation at least partially to the level of C1-S1 of the subject.

Consistent with disclosed embodiments, the neuromodulation system 10 can be a closed-loop system. In some embodiments, the neuromodulation system 10 can be an open-loop system or a pseudo-open-loop system. Consistent with disclosed embodiments, the closed-loop neuromodulation system 10 can include a sensor 300. The sensor 300 of such a closed-loop neuromodulation system can be or include at least one of an IMU, accelerometer, pressure sensor, motion capture sensor, force plate, rehabilitation device, electrode, electrode lead, infrared camera, GPS, smartphone, smartwatch, action-camcorder, video processing, virtual reality module, magnetic resonance imaging, goniometer, insole, EEG, skin resistance sensor, or infrared sensor.

Consistent with disclosed embodiments, the use of the neuromodulation system 10 can include the use of tonic neuromodulation (e.g., tonic neurostimulation). In some instances, tonic neuromodulation can be applied at a frequency between 1 and 125 Hz. In some embodiments, the system can provide frequency neuromodulation on top of a carrying frequency. In particular, a frequency of the carrying frequency can be between 1 and 10 kHz, preferably between 5 and 10 kHz. In various embodiments, the neuromodulation system can be configured to use burst and/or high-frequency neuromodulation (e.g., neurostimulation).

Consistent with disclosed embodiments, the neuromodulation system 10 can be used post-implantation of the neuromodulation system 10. In various embodiments, the neuromodulation system can be used throughout rehabilitation of the subject and/or during at least one of stabilization in bed, mobilization in bed, mobilization at bedside, standing, stepping, balance training, locomotor training, fluent locomotion, pre-surgery, post-surgery, post-setting of neuromodulation system 10, spinal shock and/or daily life activities.

Consistent with disclosed embodiments, the system can be used indoors. In some embodiments, such indoor use can include use in least one of clinics, gym, physiotherapy room, occupational room, aquatic room, sport activity room, recreation facility, restaurant, cinema, bathroom, bedroom, kitchen, living room, or similar indoor environments. In various embodiments, the neuromodulation system can be used outdoors. Consistent with disclosed embodiments, the neuromodulation system 10 can be used during activities performed by the subject.

Consistent with disclosed embodiments, the neuromodulation system 10 can be used or operated by at least one person. In some embodiments, the neuromodulation system 10 can be used by the subject; or by at least one of a family member, a friend, a therapist, a physiotherapist, a nurse, an electrophysiologist, a non-professional user, a professional user, an occupational therapist, a surgeon, a technician (clinical/ FCE), and/or a (treating) physician of the subject.

### Thermoregulation system

Consistent with disclosed embodiments, the neuromodulation system 10 can be used for the treatment and/or modulation of at least one condition and/or function and/or dysfunction of a subject's thermoregulation system. In some embodiments, the at least one condition and/or function and/or dysfunction can include cutaneous vascularization. In various embodiments, the at least one condition could additionally and/or alternatively include at least one of blood volume, thermoregulation, lipogenesis/lipolysis imbalance, sweat glands, or lipid metabolism.

Consistent with disclosed embodiments, the at least one abnormal body function and/or disease can be spinal cord injury. In various embodiments, the at least one abnormal body function and/or disease can be or include at least one of tumor, obesity, trauma, stroke, damaged dorsal root, or hyperthyroidism.

Consistent with disclosed embodiments, treating the subject can include treating the spinal cord of the subject. In various embodiments, treating the subject can include treating at least one of sympathetic and/or parasympathetic nervous system, dorsal root to muscle, muscle, smooth muscle and/or nerve, preferably in the brain (e.g., in the thalamus and/or hypothalamus) of the subject.

In some embodiments, the neuromodulation system 10 can be used during spinal shock. In various embodiments, the neuromodulation system 10 can be used throughout rehabilitation of the subject and/or during at least one of stabilization in bed, mobilization in bed, mobilization at bedside, verticalization, blood pressure lowering, blood pressure rehabilitation, blood pressure stabilization, cardiac rehabilitation, cardiac stabilization, standing, stepping, balance training, locomotor training, fluent locomotion, pre-surgery, post-surgery, pre-implantation, post-implantation, post-setting of neuromodulation system and/or daily life activities.

Consistent with disclosed embodiments, the neuromodulation system 10 can be configured to use together with an assistive device.

Consistent with disclosed embodiments, neuromodulation system can include a neurostimulation system 10. The neurostimulation system 10 can be or include at least one of an electrical neurostimulation system, a mechanical neurostimulation system, or a pharmacological neurostimulation system. In some embodiments, an electrical neurostimulation system can include an epidural electrical stimulation system. In various embodiments, the electrical neurostimulation system can be or include at least one of transcutaneous electrical stimulation system, dorsal root ganglion stimulation system, intramuscular stimulation system, or subdural stimulation system.

Consistent with disclosed embodiments, the electrical stimulation system can be used to provide stimulation at least partially to the level of T7 of the subject. In various embodiments, the electrical stimulation system can be used to provide stimulation at least partially to the brain of the subject (e.g., to the hypothalamus of the subject).

Consistent with disclosed embodiments, the neuromodulation system 10 can be a closed-loop system. In some embodiments, the neuromodulation system 10 can be an open-loop system or a pseudo-open-loop system. Consistent with disclosed embodiments, the closed-loop neuromodulation system can include a sensor 300. The sensor 300 can be or include at least one of a thermometer (e.g., a skin thermometer or implanted thermometer), an electrode, electrode lead, or other type of sensor.

Consistent with disclosed embodiments, the neuromodulation system can be configured to use burst neuromodulation (e.g., burst neurostimulation). In some embodiments, the neuromodulation system 10 can be configured to apply burst neuromodulation comprising pairs, triplets, quadruplets, quintuplets of pulses. The pulses can have a burst frequency of at least 200 Hz, preferably 300 to 750 Hz. The pulses can have an interburst frequency of between 1 and 125 Hz, preferably between 10 and 60 Hz.

Consistent with disclosed embodiments, the use of the neuromodulation system 10 can include the use of tonic neuromodulation (e.g., tonic neurostimulation). In some embodiments, the system 10 can provide frequency neuromodulation on top of a carrying frequency.

Consistent with disclosed embodiments, the system 10 can be used indoors. In some embodiments, such indoor use can include use in least one of clinics, gym, physiotherapy room, occupational room, aquatic room, sport activity room, recreation facility, restaurant, cinema, bathroom, bedroom, kitchen, living room, or similar indoor environments. In various embodiments, the neuromodulation system 10 can be used outdoors. Consistent with disclosed embodiments, the neuromodulation system can be used during activities performed by the subject.

Consistent with disclosed embodiments, the neuromodulation system 10 can be used or operated by at least one person. In some embodiments, the neuromodulation system 10 can be used by the subject; or by at least one of a family member, a friend, a therapist, a physiotherapist, a nurse, an electrophysiologist, a non-professional user, a professional user, an occupational therapist, a surgeon, a technician (clinical/ FCE), and/or a (treating) physician of the subject.

Interactions between the programable device 100 and the neurostimulation device 200.

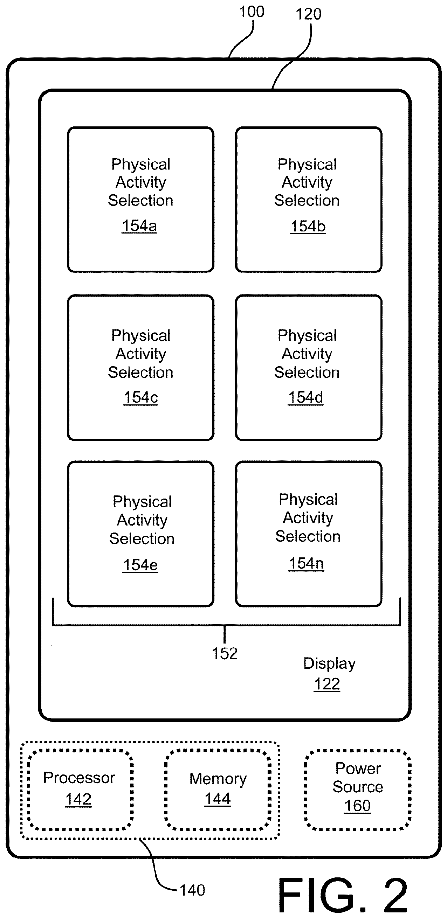

Fig. 2 shows the programable device 100. While the programable device 100 is depicted in Fig. 2 as a smart phone, any suitable computer system may be used. For example, the programable device 100 may be a general-purpose computer, a personal computer, a workstation, a mainframe computer, a notebook, a global positioning device, a laptop computer, a smart phone, a smart watch, a tablet, a personal digital assistant, a network server, and any other electronic device that may interact with a user to develop programming code.

Although not shown, the various components of the programable device 100 need not be fully contained within the programable device 100. Each of the components may be physically separated from another and more than one of the components may be used to perform methods consistent with the present invention. Even though the components may be physically separated, the components may still be communicably connected by means of wired or wireless technology. For example, different components of system 10 and programable device 100 may be connected through the Internet, a LAN (local area network), a WAN (wide area network), databases, servers, RF (radio frequency) signals, cellular technology, Ethernet, telephone, "TCP/IP" (transmission control protocol/internet protocol), and any other electronic communication format.

The basic components of programable device 100 include a user interface 120 (having a display device 122), a controller 140 (having a processor 142 and a memory device 144), and a power source 160 (e.g., batteries), although programable device 100 may contain other components including those components that facilitate electronic communication. The user interface 120 may include devices such as an input and output devices (not shown). Programable device 100 may include computer hardware components such as a combination of Central Processing Units (CPUs) or processors, buses, memory devices, storage units, data processors, input devices, output devices, network interface devices, and other types of components that will become apparent to those skilled in the art. Programable device 100 may further include application programs that may include software modules, sequences of instructions, routines, data structures, display interfaces, and other types of structures that execute operations of the present invention.

One of the hardware components in the programable device 100 is processor 142. Processor 142 may be an ASIC (Application Specific Integrated Circuit) or it may be a general-purpose processor. Processor 142 may include more than one processor. For example, processors may be situated in parallel, series, or both in order to process all or part of the computer instructions that are to be processed.

Memory device 144 may include all forms of computer-readable storage mediums such as non-volatile or volatile memories including, by way of example, semiconductor memory devices, such as EPROM, RAM, ROM, DRAM, EEPROM, and flash memory devices; magnetic disks such as internal hard disks and removable disks; magneto-optical disks; DVD disks, and CD-ROM disks. Memory device 144 may be used to store program code 150.

The program code 150 is the software part of the programable device 100 that provides and facilitates functionality to allow the patient 50 to monitor and control his therapy and provide information to his Rehab Professional. The program code could be in the form of, for example: (i) firmware; (ii) a mobile device app; (iii) a general computer program; (iv) an interactive website or web-app; (v) etc.

Display device 122 may be any conventional user interface display device. For example, display device 122 may include computer monitors, televisions, and LCD displays. Display device 120 may display GUI (Graphical User Interface) 152 which allows a user to interact with programable device 100 hardware and software applications embodied in programable device 100.

As seen in Fig. 2, when the program code 150 is executed, the GUI 152 will display a series of physical activities 154a-n that the user 50 may wish to perform and can select. These physical activities may include: (i) specific independent rehabilitation training regimens (e.g., standing, sitting; walking, using stairs, arm movements, etc.) (ii) specific household tasks in his personal environment (e.g., cooking, cleaning, eating, etc.); (iii) controlling blood pressure (raising or lowering); (iv) voiding of bladder; (v) voiding of bowels; (vi) suppression of spasticity; (vii) sexual activity; (viii) suppression of pain; or (ix) any of the other activities or control of conditions described above that the neuromodulator 200 can influence.

The GUI 152 may display only certain physical activities at any one time based on the needs of the patient 50 or suggestions of the healthcare provider 450. For example, a patient may initially only be able to choose between standing and sitting at a first intensity level. Based on performance feedback from the sensors 300, the programable device 100 may then offer standing and sitting at a second intensity level. Additionally or alternatively, the healthcare provider 450, after reviewing the performance feedback at the workstation 400, may send a signal to the programable device 100 so that it offers a new walking program.

As described above, the user interface 120 may include input and output devices. While Fig. 2 depicts touchscreen that acts as both the display device 122 and in input device, other inputs and outputs may be utilized instead or in addition. For example, another input device could be a microphone allowing for voice-controlled input. The programable device 100 could support audio input in the form of verbal messages (e.g., "I want to walk") or specific other sounds that could trigger events. Another example input device could be a camera and/or an accelerometer and/or gyroscope that is capable of capturing physical movement events. As such, these sensors could be used to register a flick of the wrist or other type of gesture. This gesture could then be used to control therapy. For example, a user 50 rotating the wrist back and forth 2 times, could trigger a stop of stimulation, without the patient having to touch a screen or use buttons. Other input devices include buttons, keyboard, etc.

Additionally, another example output device could be a speaker. Instead of having to glance at a screen 122, the patient could be informed of events by means of audio. This could include, for example: (i) a countdown mechanism, where the patient 50 is informed when stimulation starts (i.e. five, four, three...); (ii) unexpected events that could compromise neurostimulation (e.g., a battery that is nearing depletion that the patient 50 may not be aware off, loss of connection quality between devices that could lead to a loss of neurostimulation, etc.); (iii) expected events that report on the results of neurostimulation; etc. Another example output device could be a haptic feedback device. Similar to audio, vibration could also be used to, for example provide a countdown, or indicate expected/unexpected events. The patient 50 may sense vibration while positioning the programable device 100 on, or near to, his body without having to visually engage with the other portions of the user interface 120, such as the screen 122.

The programmable device allows a patient could be provided access to reviewing and controlling (limited) stimulation properties, such as a holistic 'stimulation volume' or 'stimulation intensity' setting (which translates to a relative current amplitude and frequency). In some arrangements, the patient 50 cannot only fine-tune his own therapy in unsupervised settings with the device 100, but this information may also be fed back to the provider 450 that can use this valuable information to further tailor the patient's therapy.

As mentioned above, the neuromodulation system 10 could experience unexpected events or errors. The programable device 100, in addition to providing control of stimulation and therapy, may also provide insight for the patient 50 into his system's 10 performance. Accordingly, the programable device 100 can be configured to, for example: (i) display results of internal software checks and inform the user 50 if any warnings/errors were flagged by devices in the system 10; (ii) provide results of electrical tests that the system 10 performs (e.g. impedance measurements); (iii) indicate the health and/or remaining lifetime of the rechargeable batteries; (iv) etc. These unexpected events or errors may also be communicated to the healthcare provider 450 at the workstation 400 by the programable device 100. This could allow the user 50 to receive assistance

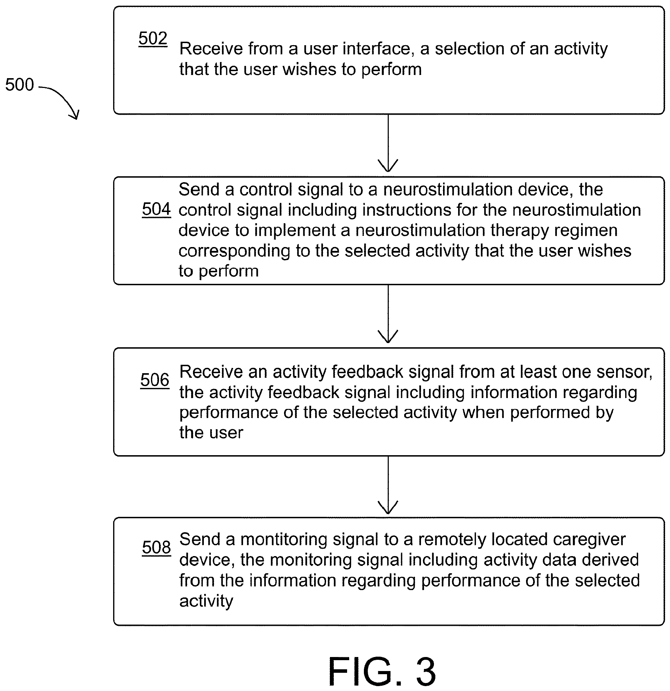

Fig. 3 shows a flowchart of the basic operations of the programable device 100 when used in the system 10.

To achieve a good therapy outcome (i.e., restoring quality-of-life) and promote neural regrowth and much as possible, the patient 50 should engage in physical training frequently. However, locations, such as a rehabilitation center, clinic, or other types of supervised environments in which the patient 50 trains may not always be available. Accordingly, a patient 50 should also be prompted to train in an unsupervised manner. This could be at home (or any place outside of a clinical context), or otherwise also at the rehabilitation center, yet without direct support from a rehabilitation professional.

The programable device 100 could be used by the patient 50 to control the stimulation of his therapy and allow him to not only train more frequently, but also to do so independently (e.g., when supervised therapy is nearing its end).

Specific types of neurostimulation therapy could be needed at moments of the day that the patient is not in a supervised environment (e.g. at the rehabilitation center) where specialized equipment that can control the therapy is present.

As such, the patient 50 can utilize the programable device 100 to allow him to start/stop and adjust basic parameters on therapy programs needed during specific moments of the day and in his own environment (e.g. home). Specific therapy programs could correspond to, for example, when the patient 50: (i) wants to perform independent rehabilitation training; (ii) needs to perform specific household tasks in his personal environment; (iii) needs to control his blood pressure; (iv) wants to void his bladder; (v) wants to void his bowels; (vi) experiences spasticity and wants to suppress this; (vii) wants to improve his sexual functions; (viii) experiences pain and wants to suppress this; or (ix) wants to initiate any of the other activities/conditions described above that the neuromodulator 200 can influence.

Fig. 3 is a flowchart of an exemplary method 500 describing operation of the controller 140 in use.

In step 502, the controller 140 receives a physical activity selection from the user interface 120.

In step 504, the controller 140 sends a control signal to a neurostimulation device 200, the control signal including instructions for the neurostimulation device 200 to implement a neurostimulation therapy regimen corresponding to the selected physical activity that the user wishes to perform.

In step 506, the controller 140 receives an activity feedback signal from at least one sensor 300, the activity feedback signal including information regarding performance of the selected physical activity when performed by the user 50.

The programable device 100 registers and logs the activities the patient 50 performs autonomously. This registration / logging may be based on the following data (or an aggregation of that data): (i) physical activities that are selected and started on the programable device 100; (ii) stimulation events registered by the neuromodulator 200 that indicate actual stimulation was applied; (iii) sensor 300 data measured by the programable device 100, indicating that the device 100 was physically used for activity; (iv) other sensor 300 data implemented in the programable device 100 (e.g., heartrate (elevation), transpiration (galvanic skin response), etc.). Data aggregation may be assisted by artificial intelligence (e.g., machine learning, symbolic, deep learning, Bayesian networks, evolutionary algorithms) derived algorithms to generate models or suggestions for training configurations and/or other physical activities to perform.

In step 508, the controller 140 sends a monitoring signal to a remotely located caregiver device 400, the monitoring signal including activity data derived from the information regarding performance of the selected physical activity.

The above described registered/aggregated data can be retrieved from the patient's 50 programable device 100 and collected in a digital dashboard (possibly a standalone application or website) that is available to the healthcare provider 450 at the health care provider workstation 400. The healthcare provider 450 may use this information to motivate the patient, optimize his/her therapy and to prevent injury. Provider 450 may thus have access to a (digital) dashboard that can present and/or visualize the data collected by the programable device 100 that is of relevance to the Provider 450. Injury may occur when strongly motivated patients over-train. The programable device 100 that registers activities performed by the patient and that keeps track of progress, allows the Rehab Professional to monitor and prevent over-training.

The foregoing descriptions have been presented for purposes of illustration. They are not exhaustive and are not limited to precise forms or embodiments disclosed. Modifications and adaptations of the embodiments will be apparent from consideration of the specification and practice of the disclosed embodiments. For example, the described implementations include hardware, but systems and methods consistent with the present disclosure can be implemented with hardware and software. In addition, while certain components have been described as being coupled to one another, such components may be integrated with one another or distributed in any suitable fashion.

Moreover, while illustrative embodiments have been described herein, the scope of the invention is defined by the appended claims.

It should be noted that, the relational terms herein such as "first" and "second" are used only to differentiate an entity or operation from another entity or operation, and do not require or imply any actual relationship or sequence between these entities or operations. Moreover, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items.

As used herein, the indefinite articles "a" and "an" mean "one or more." Similarly, the use of a plural term does not necessarily denote a plurality unless it is unambiguous in the given context. Further, since numerous modifications and variations will readily occur from studying the present disclosure, it is not desired to limit the disclosure to the exact construction and operation illustrated and described, and accordingly, all suitable modifications and equivalents may be resorted to, falling within the scope of the disclosure.

As used herein, unless specifically stated otherwise, the terms "and/or" and "or" encompass all possible combinations, except where infeasible. For example, if it is stated that a database may include A or B, then, unless specifically stated otherwise or infeasible, the database may include A, or B, or A and B. As a second example, if it is stated that a database may include A, B, or C, then, unless specifically stated otherwise or infeasible, the database may include A, or B, or C, or A and B, or A and C, or B and C, or A and B and C.

It is appreciated that the above-described embodiments can be implemented by hardware, or software (program codes), or a combination of hardware and software. If implemented by software, it may be stored in the above-described computer-readable media. The software, when executed by the processor can perform the disclosed exemplary methods. The computing units and other functional units described in this disclosure can be implemented by hardware, or software, or a combination of hardware and software. One of ordinary skill in the art will also understand that multiple ones of the above-described modules/units may be combined as one module/unit, and each of the above-described modules/units may be further divided into a plurality of submodules/ sub-units.

In the foregoing specification, embodiments have been described with reference to numerous specific details that can vary from implementation to implementation. Certain adaptations and modifications of the described embodiments can be made, provided that they fall under the scope of the appended claims.

It is also intended that the sequence of steps shown in figures are only for illustrative purposes and are not intended to be limited to any particular sequence of steps. As such, those skilled in the art can appreciate that these steps can be performed in a different order while implementing the same method.

## Claims

1. A system (10) for assisting and tracking a user with a neurological disorder, the system comprising:
a user interface (120) configured to allow the user to select an activity that the user wishes to perform;
a controller (140) configured to send a control signal to a neurostimulation device (200), the control signal including instructions for the neurostimulation device to implement a neurostimulation therapy regimen corresponding to the selected activity that the user wishes to perform,
wherein the controller is further configured to receive an activity feedback signal from at least one sensor (300) the activity feedback signal including information regarding performance of the selected activity when performed by the user; and
send a monitoring signal to a remotely located caregiver device, the monitoring signal including activity data derived from the information regarding performance of the selected activity,
wherein the activity is a rehabilitation exercise routine;
**characterized in that**
the activity data includes information regarding how much of the rehabilitation exercise routine was completed by the user.

2. The system of claim 1, wherein the activity data includes information regarding a duration for which the neurostimulation therapy regimen has been applied.

3. The system of claim 1 or claim 2,
wherein a sensor of the at least one sensor is an accelerometer and/or gyroscope configured to attach to a portion of the user's body and detect movement information of the portion of the user's body; and/or
wherein a sensor of the at least one sensor is
(i) a pressure sensor configured to detect a hemodynamic system state of the user, or
(ii) a sensor configured to a bladder content state of the user.

4. The system of one of claims 1 to 3:
wherein the selection of the activity is chosen by the user from a list of activities;
wherein the neurostimulation therapy regimen to be implemented by the neurostimulation device is selected from a list of neurostimulation therapy regimens;
wherein each of the list of activities corresponds a different neurostimulation regimen of the list of neurostimulation therapy regimens.

5. The system of claims 1 to 4:
wherein the controller is further configured to send a patient reporting signal to the user interface, the patient reporting signal including the activity data;
wherein the user interface is further configured to communicate the activity data to the user.

6. The system of claim 5, wherein the user interface is configured to communicate the activity data to the user by auditory cues and/or auditory messaging and/or vibrational messaging.

7. The system of claim 5 or 6, wherein the patient reporting signal further includes a supplemental activity assessment, the supplemental activity assessment including messaging
(i) to encourage and motivate the user to select further activities, or
(ii) to advise the user to reduce further selections of physical of physical activities.

8. The system of claim 7, wherein the supplemental activity assessment includes messaging to encourage and motivate the user to select further physical activities at an increased intensity over the initially selected activity.

9. The system of claims 1 to 8, wherein the controller is further configured to receive a caregiver feedback signal from the remotely located caregiver device, the caregiver feedback signal including messaging to the user and/or instructions for the neurostimulation device to implement a new neurostimulation therapy regimen that the user may select.

10. The system of claim 9, wherein the caregiver feedback signal includes messaging
(i) to encourage and motivate the user to select further activities, or
(ii) to advise the user to reduce further selections of activities; and/or
wherein the caregiver feedback signal includes instructions for the neurostimulation device to implement the new neurostimulation therapy regimen that the user may select, the new neurostimulation therapy being unavailable for selection by the user prior to the receiving of the caregiver feedback signal.

11. The system of claim 9 or 10:
wherein the controller is further configured to receive a device status signal from the neurostimulation device, the device status signal including error information regarding operating errors of the neurostimulation device;
wherein the user interface is further configured to communicate the error information to the user.

12. The system of claim 11, wherein the controller is further configured to send a monitoring signal to a remotely located caregiver device, the monitoring signal including activity data derived from the information regarding performance of the selected activity;
wherein the monitoring signal further includes the error information.

13. The system of one of claims 1 to 12, wherein the control signal is configured to implement the neurostimulation therapy regimen by selectively activating individual electrodes of the neurostimulation device and to control amplitude, frequency, wavelength, and polarity of neurostimulation at those individual electrodes for the neurostimulation therapy regimen corresponding to the selected activity.

14. The system of one of claims 1 to 13, wherein the user interface is configured to communicate to the user that the neurostimulation device is implementing the neurostimulation therapy regimen corresponding to the selected activity at a time that the neurostimulation device is implementing the neurostimulation therapy.

15. A computer-readable storage medium comprising instructions for assisting and tracking a user with a neurological disorder, which when executed on a computer processor causes the system according to any of claims 1-14 to perform a method, the method comprising:
receiving, from a user interface, a selection of an activity that the user wishes to perform;
sending a control signal to a neurostimulation device, the control signal including instructions for the neurostimulation device to implement a neurostimulation therapy regimen corresponding to the selected activity that the user wishes to perform,
wherein the method further comprises:
receiving an activity feedback signal from at least one sensor, the activity feedback signal including information regarding performance of the selected activity when performed by the user; and
sending a monitoring signal to a remotely located caregiver device, the monitoring signal including activity data derived from the information regarding performance of the selected activity,
wherein the activity is a rehabilitation exercise routine;
wherein the activity data includes information regarding how much of the rehabilitation exercise routine was completed by the user.

## Patentansprüche

1. System (10) zur Unterstützung und Verfolgung eines Benutzers mit einer neurologischen Störung, wobei das System umfasst:
eine Benutzerschnittstelle (120), die so konfiguriert ist, dass sie es dem Benutzer ermöglicht, eine Aktivität auszuwählen, die er ausführen möchte;
eine Steuerung (140), die dazu konfiguriert ist, ein Steuersignal an eine Neurostimulationsvorrichtung (200) zu senden, wobei das Steuersignal Anweisungen für die Neurostimulationsvorrichtung einschließt, ein Neurostimulationstherapieschema umzusetzen, das der ausgewählten Aktivität entspricht, die der Benutzer durchführen möchte,
wobei die Steuerung ferner dazu konfiguriert ist, ein Aktivitäts-Feedbacksignal von mindestens einem Sensor (300) zu empfangen, wobei das Aktivitäts-Feedbacksignal Informationen bezüglich der Durchführung der ausgewählten Aktivität einschließt, wenn sie vom Benutzer durchgeführt wird; und
Senden eines Überwachungssignals an eine entfernt angeordnete Pflegevorrichtung, wobei das Überwachungssignal Aktivitätsdaten einschließt, die aus den Informationen bezüglich der Durchführung der ausgewählten Aktivität abgeleitet wurden,
wobei die Aktivität ein Rehabilitationsübungsprogramm ist;
**dadurch gekennzeichnet, dass** die Aktivitätsdaten Informationen darüber einschließen, wie viel von dem Rehabilitationsübungsprogramm von dem Benutzer abgeschlossen wurde.

2. System nach Anspruch 1, wobei die Aktivitätsdaten Informationen hinsichtlich der Dauer einschließen, für die das Neurostimulationstherapieschema angewendet wurde.

3. System nach Anspruch 1 oder Anspruch 2,
wobei ein Sensor des mindestens einen Sensors ein Beschleunigungsmesser und/oder Gyroskop ist, der/das so konfiguriert ist, dass er/es an einem Körperteil des Benutzers angebracht wird und Bewegungsinformationen dieses Körperteils des Benutzers erkennt; und/oder
wobei ein Sensor des mindestens einen Sensors
(i) ein Drucksensor ist, der dazu konfiguriert ist, einen hämodynamischen Systemzustand des Benutzers zu erfassen, oder
(ii) ein Sensor ist, der für die Erfassung des Blaseninhalts des Benutzers konfiguriert ist.

4. System nach einem der Ansprüche 1 bis 3:
wobei die Auswahl der Aktivität durch den Benutzer aus einer Liste von Aktivitäten erfolgt;
wobei das von der Neurostimulationsvorrichtung durchzuführende Neurostimulationstherapieschema aus einer Liste von Neurostimulationstherapieschemata ausgewählt wird;
wobei jede der Aktivitätenlisten einem anderen Neurostimulationsschema aus der Liste der Neurostimulationstherapieschemata entspricht.

5. System nach Anspruch 1 bis 4:
wobei die Steuerung ferner dazu konfiguriert ist, ein Patientenmeldesignal an die Benutzerschnittstelle zu senden, wobei das Patientenmeldesignal die Aktivitätsdaten einschließt;
wobei die Benutzerschnittstelle ferner dazu konfiguriert ist, dem Benutzer die Aktivitätsdaten mitzuteilen.

6. System nach Anspruch 5, wobei die Benutzerschnittstelle dazu konfiguriert ist, dem Benutzer die Aktivitätsdaten durch akustische Signale und/oder akustische Nachrichten und/oder Vibrationsnachrichten mitzuteilen.

7. System nach Anspruch 5 oder 6, wobei das Patientenmeldesignal ferner eine ergänzende Aktivitätsbewertung einschließt, wobei die ergänzende Aktivitätsbewertung Nachrichten einschließt, um
(i) den Benutzer zu ermutigen und zu motivieren, weitere Aktivitäten auszuwählen, oder
(ii) dem Benutzer zu raten, die Auswahl weiterer körperlicher Aktivitäten zu reduzieren.

8. System nach Anspruch 7, wobei die ergänzende Aktivitätsbewertung Nachrichten einschließt, um den Benutzer zu ermutigen und zu motivieren, weitere körperliche Aktivitäten mit einer höheren Intensität als die ursprünglich ausgewählte Aktivität auszuwählen.

9. System nach den Ansprüchen 1 bis 8, wobei die Steuerung ferner dazu konfiguriert ist, ein Pfleger-Feedbacksignal von der entfernt angeordneten Pflegervorrichtung zu empfangen, wobei das Pfleger-Feedbacksignal Nachrichten an den Benutzer und/oder Anweisungen für die Neurostimulationsvorrichtung zur Umsetzung eines neuen, vom Benutzer ausgewählten Neurostimulationstherapieschemas umfasst.

10. System nach Anspruch 9, wobei das Pfleger-Feedbacksignal Nachrichten einschließt, um
(i) den Benutzer zu ermutigen und zu motivieren, weitere Aktivitäten auszuwählen, oder
(ii) dem Benutzer zu empfehlen, die Auswahl weiterer Aktivitäten einzuschränken; und/oder
wobei das Pfleger-Feedbacksignal Anweisungen für die Neurostimulationsvorrichtung zur Umsetzung des neuen, vom Benutzer ausgewählten Neurostimulationstherapieschemas einschließt, wobei die neue Neurostimulationstherapie vor dem Empfang des Pfleger-Feedbacksignals für die Auswahl durch den Benutzer nicht verfügbar ist.

11. System nach Anspruch 9 oder 10:
wobei die Steuerung ferner dazu konfiguriert ist, ein Vorrichtungdsstatussignal von der Neurostimulationsvorrichtung zu empfangen, wobei das Vorrichtungsstatussignal Fehlerinformationen bezüglich Betriebsfehlern der Neurostimulationsvorrichtung umfasst;
wobei die Benutzerschnittstelle ferner dazu konfiguriert ist, dem Benutzer die Fehlerinformationen mitzuteilen.

12. System nach Anspruch 11, wobei die Steuerung ferner dazu konfiguriert ist, ein Überwachungssignal an eine entfernt angeordnete Pflegervorrichtung zu senden, wobei das Überwachungssignal Aktivitätsdaten einschließt, die aus den Informationen bezüglich der Durchführung der ausgewählten Aktivität abgeleitet wurden;
wobei das Überwachungssignal ferner die Fehlerinformationen einschließt.

13. System nach einem der Ansprüche 1 bis 12, wobei das Steuersignal dazu konfiguriert ist, das Neurostimulationstherapieschema durch selektive Aktivierung einzelner Elektroden der Neurostimulationsvorrichtung umzusetzen und Amplitude, Frequenz, Wellenlänge und Polarität der Neurostimulation an diesen einzelnen Elektroden für das Neurostimulationstherapieschema entsprechend der ausgewählten Aktivität zu steuern.

14. System nach einem der Ansprüche 1 bis 13, wobei die Benutzerschnittstelle dazu konfiguriert ist, dem Benutzer mitzuteilen, dass die Neurostimulationsvorrichtung das der ausgewählten Aktivität entsprechende Neurostimulationstherapieschema zu einem Zeitpunkt durchführt, zu dem die Neurostimulationsvorrichtung die Neurostimulationstherapie durchführt.

15. Computerlesbares Speichermedium, umfassend Anweisungen zur Unterstützung und Verfolgung eines Benutzers mit einer neurologischen Störung, die, wenn sie auf einem Computerprozessor ausgeführt werden, bewirken, dass das System nach einem der Ansprüche 1 bis 14 ein Verfahren ausführt, wobei das Verfahren umfasst:
Empfangen einer Auswahl einer Aktivität, die der Benutzer ausführen möchte, von einer Benutzerschnittstelle;
Senden eines Steuersignals an eine Neurostimulationsvorrichtung, wobei das Steuersignal Anweisungen für die Neurostimulationsvorrichtung einschließt, ein Neurostimulationstherapieprogramm durchzuführen, das der ausgewählten Aktivität entspricht, die der Benutzer durchführen möchte,
wobei das Verfahren ferner umfasst:
Empfangen eines Aktivitäts-Feedbacksignals von mindestens einem Sensor, wobei das Aktivitäts-Feedbacksignal Informationen bezüglich der Durchführung der ausgewählten Aktivität einschließt, wenn diese vom Benutzer ausgeführt wird; und
Senden eines Überwachungssignals an eine entfernt angeordnete Pflegervorrichtung, wobei das Überwachungssignal Aktivitätsdaten einschließt, die aus den Informationen bezüglich der Durchführung der ausgewählten Aktivität abgeleitet wurden,
wobei die Aktivität ein Rehabilitationsübungsprogramm ist;
wobei die Aktivitätsdaten Informationen darüber einschließen, wie viel von dem Rehabilitationsübungsprogramm von dem Benutzer abgeschlossen wurde.

## Revendications

1. Système (10) d'assistance et de suivi d'un utilisateur souffrant d'un trouble neurologique, le système comprenant :
une interface utilisateur (120) configurée pour permettre à l'utilisateur de sélectionner une activité que l'utilisateur souhaite effectuer ;
un dispositif de commande (140) configuré pour envoyer un signal de commande à un dispositif de neurostimulation (200), le signal de commande comportant des instructions pour que le dispositif de neurostimulation mette en oeuvre un régime de thérapie par neurostimulation correspondant à l'activité sélectionnée que l'utilisateur souhaite effectuer,
dans lequel le dispositif de commande est en outre configuré pour recevoir un signal de rétroaction d'activité à partir d'au moins un capteur (300), le signal de rétroaction d'activité comportant des informations relatives à l'exécution de l'activité sélectionnée lorsqu'elle est effectuée par l'utilisateur ; et
envoyer un signal de surveillance à un dispositif de soins situé à distance, le signal de surveillance comportant des données d'activité dérivées des informations relatives à l'exécution de l'activité sélectionnée,
dans lequel l'activité est une routine d'exercices de rééducation ;
**caractérisé en ce que** les données d'activité comportent des informations relatives à la quantité d'exercices de rééducation effectués par l'utilisateur.

2. Système selon la revendication 1, dans lequel les données d'activité comportent des informations relatives à une durée pendant laquelle le régime de thérapie par neurostimulation a été appliqué.

3. Système selon la revendication 1 ou la revendication 2,
dans lequel un capteur de l'au moins un capteur est un accéléromètre et/ou un gyroscope configuré pour se fixer sur une partie du corps de l'utilisateur et détecter des informations sur les mouvements de la partie du corps de l'utilisateur ; et/ou
dans lequel un capteur de l'au moins un capteur est
(i) un capteur de pression configuré pour détecter un état du système hémodynamique de l'utilisateur, ou
(ii) un capteur configuré pour mesurer un état du contenu de la vessie de l'utilisateur.

4. Système selon l'une des revendications 1 à 3 :
dans lequel la sélection de l'activité est choisie par l'utilisateur à partir d'une liste d'activités ;
dans lequel le régime de thérapie par neurostimulation à mettre en oeuvre par le dispositif de neurostimulation est sélectionné à partir d'une liste de régimes de thérapie par neurostimulation ;
dans lequel chacune de la liste d'activités correspond à un régime de neurostimulation différent de la liste des régimes de thérapie par neurostimulation.

5. Système selon les revendications 1 à 4 :
dans lequel le dispositif de commande est en outre configuré pour envoyer un signal de rapport sur le patient à l'interface utilisateur, le signal de rapport sur le patient comportant les données d'activité ;
dans lequel l'interface utilisateur est en outre configurée pour communiquer les données d'activité à l'utilisateur.

6. Système selon la revendication 5, dans lequel l'interface utilisateur est configurée pour communiquer les données d'activité à l'utilisateur par des signaux auditifs et/ou des messages auditifs et/ou des messages vibratoires.

7. Système selon la revendication 5 ou 6, dans lequel le signal de rapport sur le patient comporte en outre une évaluation supplémentaire de l'activité, l'évaluation supplémentaire de l'activité comportant un message
(i) pour encourager et motiver l'utilisateur à sélectionner d'autres activités, ou
(ii) pour conseiller à l'utilisateur de réduire les sélections d'autres activités physiques.

8. Système selon la revendication 7, dans lequel l'évaluation supplémentaire de l'activité comporte un message visant à encourager et à motiver l'utilisateur à sélectionner d'autres activités physiques à une intensité supérieure à celle de l'activité initialement sélectionnée.

9. Système selon les revendications 1 à 8, dans lequel le dispositif de commande est en outre configuré pour recevoir un signal de rétroaction du soignant à partir du dispositif de soignant situé à distance, le signal de rétroaction du soignant comportant un message destiné à l'utilisateur et/ou des instructions pour que le dispositif de neurostimulation mette en oeuvre un nouveau régime de thérapie par neurostimulation que l'utilisateur peut sélectionner.

10. Système selon la revendication 9, dans lequel le signal de rétroaction du soignant comporte un message
(i) pour encourager et motiver l'utilisateur à sélectionner d'autres activités, ou
(ii) pour conseiller à l'utilisateur de réduire les sélections d'autres activités ; et/ou
dans lequel le signal de rétroaction du soignant comporte des instructions pour que le dispositif de neurostimulation mette en oeuvre le nouveau régime de thérapie par neurostimulation que l'utilisateur peut sélectionner, la nouvelle thérapie de neurostimulation étant indisponible pour la sélection par l'utilisateur avant la réception du signal de rétroaction du soignant.

11. Système selon la revendication 9 ou 10 :
dans lequel le dispositif de commande est en outre configuré pour recevoir un signal d'état du dispositif de neurostimulation, le signal d'état du dispositif comportant des informations d'erreur relatives aux erreurs de fonctionnement du dispositif de neurostimulation ;
dans lequel l'interface utilisateur est en outre configurée pour communiquer les informations d'erreur à l'utilisateur.

12. Système selon la revendication 11, dans lequel le dispositif de commande est en outre configuré pour envoyer un signal de surveillance à un dispositif de soignant situé à distance, le signal de surveillance comportant des données d'activité dérivées des informations relatives à l'exécution de l'activité sélectionnée ;
dans lequel le signal de surveillance comporte en outre des informations d'erreur.

13. Système selon l'une des revendications 1 à 12, dans lequel le signal de commande est configuré pour mettre en oeuvre le régime de thérapie par neurostimulation en activant sélectivement des électrodes individuelles du dispositif de neurostimulation et pour commander l'amplitude, la fréquence, la longueur d'onde et la polarité de la neurostimulation au niveau de ces électrodes individuelles pour le régime de thérapie par neurostimulation correspondant à l'activité sélectionnée.

14. Système selon l'une des revendications 1 à 13, dans lequel l'interface utilisateur est configurée pour communiquer à l'utilisateur que le dispositif de neurostimulation met en oeuvre le régime de thérapie par neurostimulation correspondant à l'activité sélectionnée à un moment où le dispositif de neurostimulation met en oeuvre la thérapie par neurostimulation.

15. Support de stockage lisible par ordinateur comprenant des instructions d'assistance et de suivi d'un utilisateur souffrant d'un trouble neurologique, qui, lorsqu'elles sont exécutées sur un processeur informatique, amènent le système selon l'une quelconque des revendications 1 à 14 à exécuter un procédé, le procédé comprenant :
la réception, à partir d'une interface utilisateur, d'une sélection d'une activité que l'utilisateur souhaite effectuer ;
l'envoie d'un signal de commande à un dispositif de neurostimulation, le signal de commande comportant des instructions pour que le dispositif de neurostimulation mette en oeuvre un régime de thérapie par neurostimulation correspondant à l'activité sélectionnée que l'utilisateur souhaite effectuer,
dans lequel le procédé comprend en outre :
la réception d'un signal de rétroaction d'activité à partir d'au moins un capteur, le signal de rétroaction d'activité comportant des informations relatives à l'exécution de l'activité sélectionnée lorsqu'elle est effectuée par l'utilisateur ; et
l'envoi d'un signal de surveillance à un dispositif de soins situé à distance, le signal de surveillance comportant des données d'activité dérivées des informations relatives à l'exécution de l'activité sélectionnée,
dans lequel l'activité est une routine d'exercices de rééducation ;
dans lequel les données d'activité comportent des informations relatives à la quantité d'exercices de rééducation effectués par l'utilisateur.
